# EUROPEAN PATENT APPLICATION

(11) **EP 1 445 254 A1**
(43) Date of publication of application: **11.08.2004**
(21) Application number: 02778097.2
(22) Date of filing: 12.11.2002
(51) Int. Cl.: C07D 231/20, C07D 417/12, C07D 413/12, C07D 401/12, C07D 413/14

(54) **PROCESS FOR PRODUCTION OF PYRAZOLE COMPOUNDS**

(30) Priority: 13.11.2001 JP 2001347985
(71) Applicant: Takeda Chemical Industries, Ltd., Osaka-shi, Osaka 541-0045 (JP)
(72) Inventor: TAWADA, Hiroyuki, Takatsuki-shi, Osaka 569-1032 (JP); YAMASHITA, Makoto, Amagasaki-shi, Hyogo 661-0012 (JP); IKEMOTO, Tomomi, Takarazuka-shi, Hyogo 665-0815 (JP); NISHIGUCHI, Atsuko, Itami-shi, Hyogo 664-0883 (JP)
(74) Representative: Rickard, Timothy Mark Adrian
(86) International application number: PCT/JP2002/011781
(87) International publication number: WO 2003/042183

(57) **Abstract**

As a method capable of conveniently producing a pyrazole compound in a high yield, which is useful as a synthetic intermediate for a pharmaceutical agent such as a therapeutic agent for diabetes and the like, a production method of a compound represented by the formula (I) 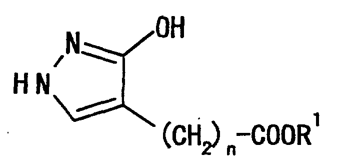 wherein n is an integer of 1 to 4 and R¹ is a hydrocarbon group optionally having substituents, or a salt thereof, is provided, which includes reacting a compound represented by the formula (II) 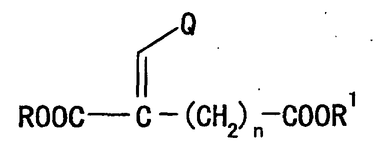 wherein Q is a hydroxy group optionally having substituents or an amino group optionally having substituents, R is a hydrocarbon group optionally having substituents and other symbols are as defined above, or a salt thereof, with hydrazine or a salt thereof.

## Description

### Technical Field

The present invention relates to a production method of a pyrazole compound useful as a synthetic intermediate for pharmaceutical agents such as a therapeutic agent for diabetes and the like, and a novel pyrazole compound used for the production method.

### Background Art

A pyrazole compound having an alkyl group substituted by an optionally esterified carboxyl group at the 4-position is known to be useful as a synthetic intermediate for pharmaceutical agents.

For example, WO01/38325 describes the following compounds 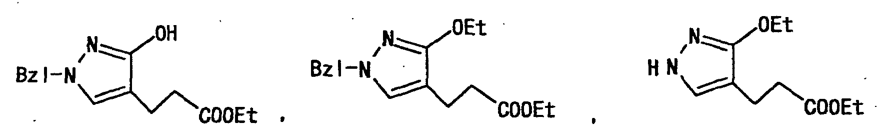 as synthetic intermediates for heterocyclic compounds useful as a therapeutic agent for diabetes and the like.

These compounds are produced by the steps of a reduction reaction, an oxidization reaction and the like using ethyl 3-hydroxy-1H-pyrazole-4-carboxylate as a starting material, during which an alkylene chain is introduced into the 4-position of pyrazole.

The above-mentioned production method using ethyl 3-hydroxy-1H-pyrazole-4-carboxylate as a starting material requires a number of steps to obtain the object substance. Therefore, provision of a production method capable of producing the object substance more conveniently is desired.

### Disclosure of the Invention

The present inventors have studied production methods of pyrazole compound having an alkyl group substituted by an optionally esterified carboxyl group at the 4-position from various aspects, and found that the object pyrazole compound can be easily produced by using, as a starting compound, a compound represented by the formula (II) 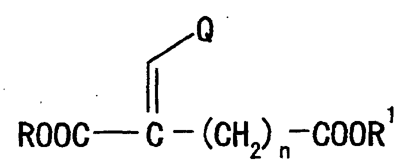 wherein Q is a hydroxy group optionally having substituents or an amino group optionally having substituents, n is an integer of 1 to 4, and R and R¹ are the same or different and each is a hydrocarbon group optionally having substituents, or a salt thereof [hereinafter sometimes to be abbreviated as compound (II)] and reacting this compound with hydrazine, which resulted in the completion of the present invention.

Accordingly, the present invention relates to
1) a production method of a compound represented by the formula (I) 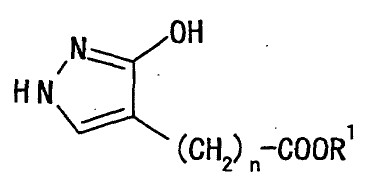 wherein the symbols in the formula are as defined above, or a salt thereof [hereinafter sometimes to be abbreviated as compound (I)], which comprises reacting compound (II) with hydrazine or a salt thereof;
2) the production method of the aforementioned 1), wherein Q is a hydroxy group;
3) the production method of the aforementioned 1), wherein n is 2;
4) the production method of the aforementioned 1), wherein the hydrocarbon group optionally having substituents for R or R¹ is an alkyl group having 1 to 8 carbon atoms;
5) compound (1);
6) a production method of a compound represented by the formula (III) 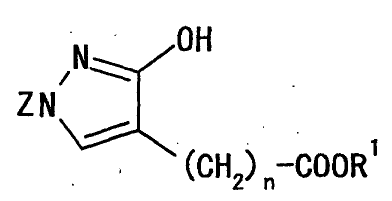 wherein Z is an acyl group and other symbols are as defined above, or a salt thereof [hereinafter sometimes to be abbreviated as compound (III)], which comprises subjecting compound (I) to an acylation reaction;
7) compound (III);
8) a production method of a compound represented by the formula (IV) 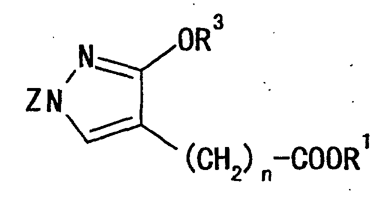 wherein R³ is an alkyl group and other symbols are as defined above, or a salt thereof [hereinafter sometimes to be abbreviated as compound (IV)], which comprises subjecting compound (III) to an alkylation reaction;
9) compound (IV);
10) a production method of a compound represented by the formula (V) 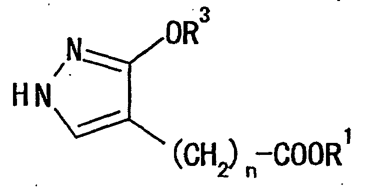 wherein the symbols in the formula are as defined above, or a salt thereof [hereinafter sometimes to be abbreviated as compound (V)], which comprises subjecting compound (IV) to a deacylation reaction;
11) a production method of a compound represented by the formula (VII) 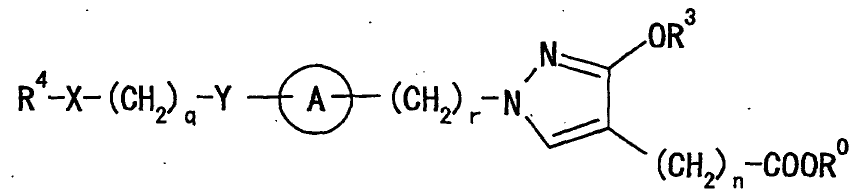 wherein R⁴ is a hydrocarbon group optionally having substituents or a heterocyclic group optionally having substituents; X is a bond, an oxygen atom, a sulfur atom, -CO-, -CS-, -CR⁵(OR⁶)- or -NR⁷- wherein R⁵ and R⁷ are each a hydrogen atom or a hydrocarbon group optionally having substituents, R⁶ is a hydrogen atom or a hydroxyl-protecting group; q is an integer of 0 to 3; Y is an oxygen atom, a sulfur atom, -SO-, -SO₂-, -NR⁸-, -CONR⁸- or -NR⁸CO- wherein R⁸ is a hydrogen atom or a hydrocarbon group optionally having substituents; ring A is an aromatic ring optionally further having 1 to 3 substituents; r is an integer of 1 to 8; R⁰ is a hydrogen atom or a hydrocarbon group optionally having substituents, and other symbols are as defined above, or a salt thereof [hereinafter sometimes to be abbreviated as compound (VII)], which comprises reacting compound (II) with hydrazine or a salt thereof to give compound (I), subjecting this compound to an acylation reaction to give compound (III), subjecting this compound to an alkylation reaction to give compound (IV), subjecting this compound to a deacylation reaction to give compound (V), reacting this compound with a compound represented by the formula (VI) 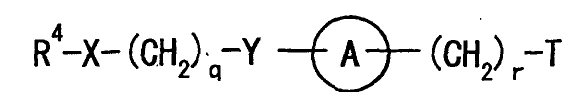 wherein T is a leaving group and other symbols are as defined above, or a salt thereof [hereinafter sometimes to be abbreviated as compound (VI)] and conducting a hydrolysis reaction where necessary;
12) a production method of compound (VII), which comprises reacting compound (II) with hydrazine or a salt thereof to give compound (I), reacting this compound with compound (VI) to give a compound represented by the formula (VIII) 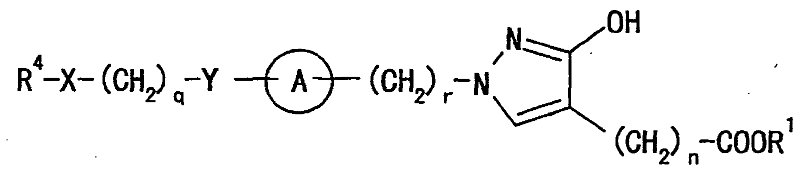
wherein the symbols in the formula are as defined above, or a salt thereof [hereinafter sometimes to be abbreviated as compound (VIII)], subjecting this compound to an alkylation reaction, and conducting a hydrolysis reaction where necessary; and the like.

The definitions of the symbols used in the present specification are described in detail in the following.

As the substituents of the "hydroxy group optionally having substituents" for Q, for example, a C₁₋₁₀ alkyl group, a C₃₋₁₀ cycloalkyl group, a C₂₋₁₀ alkenyl group, a C₃₋₁₀ cycloalkenyl group, a C₇₋₁₀ aralkyl group, a C₁₋₁₃ acyl group (preferably a C₂₋₁₀ alkanoyl group, a C₇₋₁₃ arylcarbonyl group etc.), a C₆₋₁₄ aryl group and the like can be mentioned.

As the "hydroxy group optionally having substituents", for example, a hydroxy group, a C₁₋₁₀ alkoxy group, a C₃₋₁₀ cycloalkyloxy group, a C₂₋₁₀ alkenyloxy group, a C₃₋₁₀ cycloalkenyloxy group, a C₇₋₁₀ aralkyloxy group, a C₂₋₁₃ acyloxy group, a C₆₋₁₄ aryloxy group and the like can be mentioned.

As used herein, preferable examples of the C₁₋₁₀ alkoxy group include methoxy, ethoxy, propoxy, isopropoxy, butoxy, isobutoxy, sec.-butoxy, t.-butoxy, pentyloxy, isopentyloxy, neopentyloxy, hexyloxy, heptyloxy, nonyloxy and the like.

Preferable examples of the C₃₋₁₀ cycloalkyloxy group include cyclopropoxy, cyclobutoxy, cyclopentyloxy, cyclohexyloxy and the like.

Preferable examples of the C₂₋₁₀ alkenyloxy group include allyloxy, crotyloxy, 2-pentenyloxy, 3-hexenyloxy and the like.

Preferable examples of the C₃₋₁₀ cycloalkenyloxy group include 2-cyclopentenyloxy, 2-cyclohexenyloxy and the like.

Preferable examples of the C₇₋₁₀ aralkyloxy group include phenyl-C₁₋₄ alkyloxy (e.g., benzyloxy, phenethyloxy etc.) and the like.

Preferable examples of the C₂₋₁₃ acyloxy group include C₂₋₄ alkanoyloxy (e.g., acetyloxy, propionyloxy, butyryloxy, isobutyryloxy etc.) and the like.

Preferable examples of the C₆₋₁₄ aryloxy group include phenoxy, naphthyloxy and the like.

As the "substituents" of the "amino group optionally having substituents" for Q, for example, a C₁₋₁₀ alkyl group, a C₃₋₁₀ cycloalkyl group, a C₂₋₁₀ alkenyl group, a C₃₋₁₀ cycloalkenyl group, a C₁₋₃ acyl group (preferably a C₂₋₁₀ alkanoyl group, a C₇₋₁₃ arylcarbonyl group etc.), a C₆₋₁₄ aryl group and the like can be mentioned. The number of substituents is one or two.

As the "amino group optionally having substituents", for example, an amino group, a mono- or di-C₁₋₁₀ alkyl-amino group, a mono- or di-C₃₋₁₀ cycloalkyl-amino group, a mono- or di-C₂₋₁₀ alkenyl-amino group, a mono- or di-C₃₋₁₀ cycloalkenyl-amino group, a mono- or di-C₁₋₁₃ acyl (preferably C₂₋₁₀ alkanoyl, C₇₋₁₃ arylcarbonyl etc.)-amino group, a mono- or di-C₆₋₁₄ aryl-amino group, an N-C₁₋₁₀ alkyl-N-C₆-₁₄ aryl-amino group and the like can be mentioned.

Here, preferable examples of the mono- or di-C₁₋₁₀ alkyl-amino group include methylamino, dimethylamino, ethylamino, diethylamino, propylamino, isopropylamino, diisopropylamino, dibutylamino and the like.

Preferable examples of the mono- or di-C₃₋₁₀ cycloalkyl-amino group include cyclopropylamino, cyclobutylamino, cyclohexylamino and the like.

Preferable examples of the mono- or di-C₂₋₁₀ alkenyl-amino group include diallylamino and the like.

Preferable examples of the mono- or di-C₃₋₁₀ cycloalkenyl-amino group include 2-cyclopentenylamino and the like.

Preferable examples of the mono- or di-C₁₋₁₃ acyl-amino group include acetylamino, propionylamino, benzoylamino and the like.

Preferable examples of the mono- or di-C₆₋₁₄ aryl-amino group include phenylamino and the like.

Preferable examples of the N-C₁₋₁₀ alkyl-N-C₆₋₁₄ aryl-amino group include N-methyl-N-phenylamino and the like.

Q is preferably a hydroxy group optionally having substituents, more preferably a hydroxy group.

n is an integer of 1 to 4, preferably 2.

As the "hydrocarbon group" of the "hydrocarbon group optionally having substituents" for R or R¹, for example, an aliphatic hydrocarbon group, an alicyclic hydrocarbon group, an alicyclic-aliphatic hydrocarbon group, an aromatic-aliphatic hydrocarbon group and an aromatic hydrocarbon group can be mentioned. These hydrocarbon groups preferably have 1 to 14 carbon atoms.

As the aliphatic hydrocarbon group, a C₁₋₈ aliphatic hydrocarbon group is preferable. As the aliphatic hydrocarbon group, for example, C₁₋₈ saturated aliphatic hydrocarbon groups (e.g., an alkyl group etc.) such as methyl, ethyl, propyl, isopropyl, butyl, isobutyl, sec.-butyl, t.-butyl, pentyl, isopentyl, neopentyl, hexyl, isohexyl, heptyl, octyl and the like; C₂₋₈ unsaturated aliphatic hydrocarbon groups (e.g., a C₂₋₈ alkenyl group, a C₄₋₈ alkadienyl group, a C₂₋₈ alkynyl group, a C₄₋₈ alkadiynyl group etc.) such as ethenyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 3-hexynyl, 2,4-hexadiynyl, 5-hexynyl, 1-heptynyl, 1-octynyl and the like can be mentioned.

As the alicyclic hydrocarbon group, a C₃₋₇ alicyclic hydrocarbon group is preferable. As the alicyclic hydrocarbon group, for example, C₃₋₇ saturated alicyclic hydrocarbon groups (e.g., a cycloalkyl group etc.) such as cyclopropyl, cyclobutyl, cyclopentyl, cyclohexyl, cycloheptyl and the like; C₅₋₇ unsaturated alicyclic hydrocarbon groups (e.g., a cycloalkenyl group, a cycloalkadienyl group etc.) such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl and the like can be mentioned.

As the alicyclic-aliphatic hydrocarbon group, groups wherein the above-mentioned alicyclic hydrocarbon group and an aliphatic hydrocarbon group are bonded (e.g., a cycloalkylalkyl group, a cycloalkenyl-alkyl group etc.) can be mentioned, with preference given to a C₄₋₉ alicyclic-aliphatic hydrocarbon group. As the alicyclic-aliphatic hydrocarbon group, for example, cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like can be mentioned.

As the aromatic-aliphatic hydrocarbon group, C₇₋₁₃ aromatic-aliphatic hydrocarbon groups (e.g., a C₇₋₁₃ aralkyl group, a C₈₋₁₃ arylalkenyl group etc.) are preferable. As the aromatic-aliphatic hydrocarbon group, for example, C₇₋₉ phenylalkyl such as benzyl, phenethyl, 1-phenylethyl, 1-phenylpropyl, 2-phenylpropyl, 3-phenylpropyl etc.; C₁₁₋₁₃ naphthylalkyl such as α-naphthylmethyl, α-naphthylethyl, β-naphthylmethyl, β-naphthylethyl etc.; C₈₋₁₀ phenylalkenyl such as styryl etc.; C₁₂₋₁₃ naphthylalkenyl such as 2- (2-naphthylvinyl) etc.; and the like can be mentioned.

As the aromatic hydrocarbon group, C₆₋₁₄ aromatic hydrocarbon groups (e.g., an aryl group etc.) are preferable. As the aromatic hydrocarbon group, for example, phenyl, naphthyl, anthryl, phenanthryl, acenaphthylenyl, biphenylyl and the like can be mentioned, with preference given to phenyl, 1-naphthyl, 2-naphthyl and the like.

Of the aforementioned hydrocarbon groups, an aliphatic hydrocarbon group is preferable, and a C₁₋₆ alkyl group is more preferable.

As the "substituents" of the "hydrocarbon group optionally having substituents" for R or R¹, for example, "a halogen atom", "a nitro group", "a hydroxy group optionally having substituents", "an amino group optionally having substituents", "a thiol group optionally having substituents", "a heterocyclic group optionally having substituents", "an acyl group" and the like can be mentioned. The number of substituents is, for example, 1 to 5, preferably 1 to 3.

Here, as the "halogen atom", fluorine, chlorine, bromine and iodine can be mentioned, with preference given to fluorine and chlorine.

As the "hydroxy group optionally having substituents" and "amino group optionally having substituents", those recited as the examples of the aforementioned Q can be mentioned.

As the substituents of the "thiol group optionally having substituents", for example, a C₁₋₁₀ alkyl group, a C₃₋₁₀ cycloalkyl group, a C₂₋₁₀ alkenyl group, a C₃₋₁₀ cycloalkenyl group, a C₇₋₁₀ aralkyl group, a C₁₋₁₃ acyl group (preferably a C₂₋₁₀ alkanoyl group, a C₇₋₁₃ arylcarbonyl group etc.); a C₆₋₁₄ aryl group and the like can be mentioned.

As the "thiol group optionally having substituents", for example, a thiol group, a C₁₋₁₀ alkylthio group, a C₃₋₁₀ cycloalkylthio group, a C₂₋₁₀ alkenylthio group, a C₃₋₁₀ cycloalkenylthio group, a C₇₋₁₀ aralkylthio group, a C₂₋₁₃ acylthio group, a C₆₋₁₄ arylthio group and the like can be mentioned.

Preferable examples of the C₁₋₁₀ alkylthio group include methylthio, ethylthio, propylthio, isopropylthio, butylthio, isobutylthio, sec.-butylthio, t.-butylthio, pentylthio, isopentylthio, neopentylthio, hexylthio, heptylthio, nonylthio and the like.

Preferable examples of the C₃₋₁₀ cycloalkylthio group include cyclopropylthio, cyclobutylthio, cyclopentylthio, cyclohexylthio and the like.

Preferable examples of the C₂₋₁₀ alkenylthio group include allylthio, crotylthio, 2-pentenylthio, 3-hexenylthio and the like.

Preferable examples of the C₃₋₁₀ cycloalkenylthio group include 2-cyclopentenylthio, 2-cyclohexenylthio and the like.

Preferable examples of the C₇₋₁₀ aralkylthio group include phenyl-C₁₋₄ alkylthio (e.g., benzylthio, phenethylthio etc.) and the like.

Preferable examples of the C₂₋₁₃ acylthio group include C₂₋₄ alkanoylthio (e.g., acetylthio, propionylthio, butyrylthio, isobutyrylthio etc.) and the like.

Preferable examples of the C₆₋₁₄ arylthio group include phenylthio, naphthylthio and the like.

As the heterocyclic group of the "heterocyclic group optionally having substituents", for example, a 5- to 7-membered monocyclic heterocyclic group containing, in addition to carbon atoms, 1 to 4 heteroatoms selected from oxygen, sulfur and nitrogen atoms as ring constituting atoms, and a fused heterocyclic group thereof can be mentioned. As the fused heterocyclic group, for example, a condensed group comprising such a 5- to 7-membered monocyclic heterocyclic group and a 6-membered ring containing 1 or 2 nitrogen atoms, a benzene ring or a 5-membered ring containing one sulfur atom, and the like can be mentioned.

Preferable examples of the heterocyclic group include aromatic heterocyclic groups such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyrimidinyl, pyridazinyl, pyrazinyl, triazinyl, benzofuranyl, isobenzofuranyl, benzo[b]thienyl, indolyl, isoindolyl, 1H-indazolyl, benzimidazolyl, benzoxazolyl, benzothiazolyl, 1H-benzotriazolyl, quinolyl, isoquinolyl, cinnolyl, quinazolyl, quinoxalinyl, phthalazinyl, naphthyridinyl, purinyl, pteridinyl, carbazolyl, α-carbonylyl, β-carbonylyl, γ-carbonylyl, acrydinyl, phenoxazinyl, phenothiazinyl, phenazinyl, phenoxathiinyl, thianthrenyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like; non-aromatic heterocyclic groups such as oxiranyl, azetidinyl, oxetanyl, thietanyl, pyrrolidinyl, tetrahydrofuryl, tetrahydropyranyl, morpholinyl, thiomorpholinyl, piperazinyl, pyrrolidinyl, piperidino, morpholino, thiomorpholino and the like.

As the substituents of the aforementioned "heterocyclic group optionally having substituents", for example, a C₁₋₆ alkyl group optionally substituted by 1 to 3 halogen atoms (e.g., fluorine, chlorine, bromine, iodine etc.); a C₂₋₆ alkenyl group optionally substituted by 1 to 3 halogen atoms (e.g., fluorine, chlorine, bromine, iodine etc.); a C₃₋₁₀ cycloalkyl group; C₆₋₁₄ aryl groups (e.g., phenyl, naphthyl etc.); aromatic heterocyclic groups (e.g., thienyl, furyl, pyridyl, oxazolyl, thiazolyl etc.); non-aromatic heterocyclic groups (e.g., tetrahydrofuryl, morpholino, thiomorpholino, piperidino, pyrrolidinyl, piperazinyl etc.); a C₇₋₉ aralkyl group; an amino group optionally mono- or di-substituted by a C₁₋₄ alkyl group or a C₂₋₈ acyl group (e.g., an alkanoyl group etc.); an amidino group; a C₂₋₈ alkanoyl group; a carbamoyl group optionally mono- or di-substituted by a C₁₋₄ alkyl group; a sulfamoyl group optionally mono- or di-substituted by a C₁₋₄ alkyl group; a carboxyl group; a C₂₋₈ alkoxycarbonyl group; a hydroxy group; a C₁₋₆ alkoxy group optionally substituted by 1 to 3 halogen atoms (e.g., fluorine, chlorine, bromine, iodine etc.); a C₂₋₅ alkenyloxy group optionally substituted by 1 to 3 halogen atoms (e.g., fluorine, chlorine, bromine, iodine etc.); a C₃₋₇ cycloalkyloxy group; a C₇₋₉ aralkyloxy group; C₆₋₁₄ aryloxy groups (e.g., phenyloxy, naphthyloxy etc.); a thiol group; a C₁₋₆ alkylthio group optionally substituted by 1 to 3 halogen atoms (e.g., fluorine, chlorine, bromine, iodine etc.); a C₇₋₉ aralkylthio group; a C₆₋₁₄ arylthio group (e.g., phenylthio, naphthylthio etc.); a sulfo group; a cyano group; an azide group; a nitro group; a nitroso group; halogen atoms (e.g., fluorine, chlorine, bromine, iodine) and the like can be mentioned. The number of substituents is, for example, 1 to 3.

As the "acyl group", for example, the groups represented by the formulas: -CO-R⁹, -CO-OR⁹, -CO-NR⁹R¹⁰, -CS-NR⁹R¹⁰, -SO₂-R⁹, -SO-R⁹ and -PO₃R⁹R¹⁰ wherein R⁹ and R¹⁰ are the same or different and each is a hydrogen atom, a hydrocarbon group optionally having substituents or a heterocyclic group optionally having substituents, and the like can be mentioned.

As the hydrocarbon group of the "hydrocarbon group optionally having substituents" for R⁹ or R¹⁰, for example, those recited as the examples of the aforementioned R or R¹ can be mentioned. Of those, a C₁₋₁₀ alkyl group, a C₃₋₁₀ cycloalkyl group, a C₂₋₁₀ alkenyl group, a C₃₋₁₀ cycloalkenyl group, a C₆₋₁₄ aryl group and a C₇₋₁₃ aralkyl group are preferable.

As the substituents of the "hydrocarbon group optionally having substituents", for example, aromatic heterocyclic groups (e.g., thienyl, furyl, pyridyl, oxazolyl, thiazolyl etc.); non-aromatic heterocyclic groups (e.g., tetrahydrofuryl, morpholino, thiomorpholino, piperidino, pyrrolidinyl, piperazinyl etc.); an amino group optionally mono- or di-substituted by a C₁₋₄ alkyl group or a C₂₋₈ acyl group (e.g. , an alkanoyl group etc.); an amidino group; a C₂₋₈ alkanoyl group; a carbamoyl group optionally mono- or di-substituted by a C₁₋₄ alkyl group; a sulfamoyl group optionally mono- or di-substituted by a C₁₋₄ alkyl group; a carboxyl group; a C₂₋₈ alkoxycarbonyl group; a hydroxy group; a C₁₋₆ alkoxy group optionally substituted by 1 to 3 halogen atoms (e.g., fluorine, chlorine, bromine, iodine etc.); a C₂₋₅ alkenyloxy group optionally substituted by 1 to 3 halogen atoms (e.g., fluorine, chlorine, bromine, iodine etc.); a C₃₋₇ cycloalkyloxy group; a C₇₋₉ aralkyloxy group; C₆₋₁₄ aryloxy groups (e.g., phenyloxy, naphthyloxy etc.); a thiol group; a C₁₋₆ alkylthio group optionally substituted by 1 to 3 halogen atoms (e.g., fluorine, chlorine, bromine, iodine etc.); a C₇₋₉ aralkylthio group; a C₆₋ ₁₄ arylthio group (e.g., phenylthio, naphthylthio etc.); a sulfo group; a cyano group; an azide group; a nitro group; a nitroso group; halogen atoms (e.g., fluorine, chlorine, bromine, iodine) and the like can be mentioned. The number of substituents is, for example, 1 to 3.

As the "heterocyclic group optionally having substituents" for R⁹ or R¹⁰, for example, those recited as the examples of the "substituents" of the "hydrocarbon group optionally having substituents" can be mentioned.

Preferable examples of the acyl group include a formyl group, a carboxyl group, C₂₋₁₁ alkanoyl groups (e.g., acetyl, propionyl, butyryl, isobutyryl, valeryl, isovaleryl, pivaloyl, hexanoyl, heptanoyl, octanoyl etc.), C₄₋₁₁ cycloalkylcarbonyl groups (e.g., cyclobutanecarbonyl, cyclopentanecarbonyl, cyclohexanecarbonyl, cycloheptanecarbonyl etc.), C₃₋₁₁ alkenoyl groups (e.g., crotonyl etc.), C₄₋₁₁ cycloalkenoyl groups (e.g., 2-cyclohexenecarbonyl etc.), C₇₋₁₃ arylcarbonyl groups (e.g., benzoyl etc.), C₁₋₆ alkylsulfonyl (e.g., methanesulfonyl, ethanesulfonyl etc.), C₂₋₇ alkoxycarbonyl (e.g., methoxycarbonyl, ethoxycarbonyl, propoxycarbonyl, tert-butoxycarbonyl etc.), C₇₋₁₅ arylcarbonyl (e.g., benzoyl, 1-naphthoyl, 2-naphthoyl etc.), C₇₋₁₅ aryloxycarbonyl (e.g., phenyloxycarbonyl, 1-naphthyloxycarbonyl, 2-naphthyloxycarbonyl etc.), C₈₋₁₄ aralkyloxycarbonyl (e.g., benzyloxycarbonyl, phenethyloxycarbonyl etc.) and the like.

The "hydrocarbon group optionally having substituents" for R or R¹ is preferably an aliphatic hydrocarbon group having 1 to 8 carbon atoms, more preferably an alkyl group having 1 to 8 carbon atoms. Particularly, methyl, ethyl and the like are preferable.

As the "acyl group" for Z, those recited as the examples of the aforementioned substituents of the "hydrocarbon group optionally having substituents" for R or R¹ can be mentioned.

The acyl group preferably include C₂₋₁₁ alkanoyl groups (e.g., acetyl, propionyl, butyryl, isobutyryl, valeryl, isovaleryl, pivaloyl, hexanoyl, heptanoyl, octanoyl etc.), C₇₋₁₅ arylcarbonyl groups (e.g., benzoyl etc.), C₁₋₆alkylsulfonyl (e.g., methanesulfonyl, ethanesulfonyl etc.), C₂₋₇ alkoxycarbonyl (e.g., methoxycarbonyl, ethoxycarbonyl, propoxycarbonyl, tert-butoxycarbonyl etc.), C₇₋₁₅ arylcarbonyl (e.g., benzoyl, 1-naphthoyl, 2-naphthoyl etc.), C₈₋₁₄ aralkyloxycarbonyl (e.g., benzyloxycarbonyl, phenethyloxycarbonyl etc.) and the like. Of these, C₈₋₁₄ aralkyloxycarbonyl (e.g., benzyloxycarbonyl, phenethyloxycarbonyl etc.) is preferable.

As the "alkyl group" for R³, for example, a C₁₋₁₀ alkyl group can be mentioned. Preferable examples of the C₁₋₁₀ alkyl group include methyl, ethyl, propyl, isopropyl, butyl, isobutyl, sec.-butyl, t.-butyl, pentyl, isopentyl, neopentyl, hexyl, heptyl, nonyl and the like.

The "alkyl group" is preferably a C₁₋₆ alkyl group, with preference given to methyl, ethyl, propyl, isopropyl and the like.

The alkyl group for R³ may be an aralkyl group, and as the aralkyl group, C₇₋₁₀ aralkyl groups such as benzyl, phenethyl and the like can be mentioned. Of these, benzyl is preferable.

As the "hydrocarbon group optionally having substituents" for R⁴, those recited as the examples of the aforementioned R or R¹ can be mentioned.

As the "heterocyclic group optionally having substituents" for R⁴, those recited as the examples of the substituents of the aforementioned "hydrocarbon group optionally having substituents" for R or R¹ can be mentioned.

R⁴ is preferably a heterocyclic group optionally having substituents or a cyclic hydrocarbon group optionally having substituents. R⁴ is more preferably a heterocyclic group optionally having substituents. Here, as the heterocyclic group, 5- or 6-membered aromatic heterocyclic groups (preferably furyl, thienyl, pyridyl, pyrimidinyl, pyrazinyl, oxazolyl, thiazolyl, triazolyl, oxadiazolyl, pyrazolyl) optionally condensed with a benzene ring are preferable. Of these, furyl, thienyl, pyridyl, pyrimidinyl, pyrazinyl, oxazolyl, thiazolyl, oxadiazolyl, benzoxazolyl, benzothiazolyl, quinolyl, pyrazolyl and the like are preferable.

Preferable examples of the substituents that the aforementioned heterocyclic group or cyclic hydrocarbon group may have include
1) furyl, thienyl, pyridyl, pyrazinyl, phenyl and naphthyl, each optionally having 1 to 3 substituents selected from a C₁₋₆ alkyl group optionally substituted by 1 to 3 halogen atoms (e.g., fluorine, chlorine, bromine, iodine etc.),
   a C₁₋₆ alkoxy group optionally substituted by 1 to 3 halogen atoms (e.g., fluorine, chlorine, bromine, iodine etc.),
   a halogen atom (e.g., fluorine, chlorine, bromine, iodine etc.),
   a nitro group,
   a hydroxy group and
   an amino group;
2) a C₁₋₆ alkyl group or a C₃₋₇ cycloalkyl group, each optionally having 1 to 3 substituents selected from
   a C₁₋₆ alkoxy group optionally substituted by 1 to 3 halogen atoms (e.g., fluorine, chlorine, bromine, iodine etc.),
   a halogen atom (e.g., fluorine, chlorine, bromine, iodine etc.),
   a nitro group,
   a hydroxy group and
   an amino group; and the like. The number of substituents is, for example, 1 or 2.

R⁴ is particularly preferably pyridyl, oxazolyl, thiazolyl, triazolyl or pyrazolyl, each optionally having 1 or 2 substituents selected from a C₁₋₃ alkyl group, a C₃₋₇ cycloalkyl group, furyl, thienyl, pyridyl, phenyl and naphthyl.

X is a bond, an oxygen atom, a sulfur atom, -CO-, -CS-, -CR⁵(OR⁶)- or -NR⁷- (R⁵ and R⁷ are each a hydrogen atom or a hydrocarbon group optionally having substituents, R⁶ is a hydrogen atom or a hydroxyl-protecting group).

As the "hydrocarbon group optionally having substituents" for R⁵ or R⁷, those recited as the examples of the aforementioned R or R¹ can be mentioned. The "hydrocarbon group optionally having substituents" preferably include C₁₋₆ alkyl groups (e.g., methyl, ethyl, propyl, isopropyl, butyl, isobutyl, sec.-butyl, t.-butyl etc.) optionally having substituents. The alkyl group may have 1 to 3 substituents at substitutable positions, and as such substituents, for example, halogen atoms (e.g., fluorine, chlorine, bromine, iodine), C₁₋₆ alkoxy groups (e.g., methoxy, ethoxy, propoxy, isopropoxy, butoxy, isobutoxy, sec.-butoxy, t.-butoxy etc.), a hydroxy group, a nitro group, an amino group, C₁₋₆ acyl groups (e.g., C₁₋₆ alkanoyl groups such as formyl, acetyl, propionyl etc.) and the like can be mentioned.

R⁵ and R⁷ are each preferably a hydrogen atom or a C₁₋₆ alkyl group.

As the "hydroxyl-protecting group" for R⁶, for example, C₁₋₆ alkyl (e.g., methyl, ethyl, propyl, isopropyl, butyl, tert-butyl etc.), phenyl, trityl, C₇₋₁₀ aralkyl (e.g., benzyl etc.), formyl, C₁₋₆ alkyl-carbonyl (e.g., acetyl, propionyl etc.), benzoyl, C₇₋₁₀ aralkyl-carbonyl (e.g., benzylcarbonyl etc.), 2-tetrahydropyranyl, 2-tetrahydrofuranyl, silyl (e.g., trimethylsilyl, triethylsilyl, dimethylphenylsilyl, tertbutyldimethylsilyl, tert-butyldiethylsilyl etc.), C₂₋₆ alkenyl (e.g., 1-allyl etc.) and the like can be mentioned. These groups are optionally substituted by 1 to 3 halogen atoms (e.g., fluorine, chlorine, bromine, iodine etc.), C₁₋₆ alkyl (e.g., methyl, ethyl, propyl etc.), C₁₋₆ alkoxy (e.g., methoxy, ethoxy, propoxy etc.), nitro and the like.

X is preferably a bond, -CR⁵(OR⁶)- or -NR⁷- (the symbol is as defined above), more preferably a bond or -NR⁷- (the symbol is as defined above). X is particularly preferably a bond.

q is an integer of 0 to 3, preferably an integer of 1 to 3, more preferably 1 or 2.

Y is an oxygen atom, a sulfur atom, -SO-, -SO₂-, -NR⁸-, -CONR⁸- or -NR⁸CO- (R⁸ is a hydrogen atom or a hydrocarbon group optionally having substituents), preferably, an oxygen atom, a sulfur atom, -NR⁸- or -NR⁸CO- (the symbol is as defined above).

Here, as the "hydrocarbon group optionally having substituents" for R⁸, the aforementioned "hydrocarbon group optionally having substituents" recited as the example of R⁵ or R⁷ can be mentioned. R⁸ is preferably a hydrogen atom.

Y is particularly preferably an oxygen atom.

As the "aromatic ring" of the "aromatic ring optionally further having 1 to 3 substituents" for ring A, for example, a benzene ring, a condensed aromatic hydrocarbon ring, a 5- or 6-membered aromatic heterocyclic ring, a condensed aromatic heterocyclic ring and the like can be mentioned.

Here, as the "condensed aromatic hydrocarbon ring", for example, a condensed aromatic hydrocarbon having 9 to 14 carbon atoms and the like can be mentioned. Concretely, naphthalene, indene, fluorene, anthracene and the like can be mentioned.

As the "5- or 6-membered aromatic heterocyclic ring", for example, a 5- or 6-membered aromatic heterocyclic ring containing, in addition to carbon atoms, 1 to 3 heteroatoms selected from nitrogen, sulfur and oxygen atoms and the like can be mentioned. Concretely, thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, furazan and the like can be mentioned.

As the "condensed aromatic heterocyclic ring", for example, a 9- to 14-membered (preferably 9- or 10-membered) condensed aromatic heterocyclic ring containing, in addition to carbon atoms, 1 to 4 heteroatoms selected from nitrogen, sulfur and oxygen atoms can be mentioned. Specifically, benzofuran, benzothiophene, benzimidazole, benzoxazole, benzothiazole, benzisothiazole, naphtho[2,3-b]thiophene, isoquinoline, quinoline, indole, quinoxaline, phenanthridine, phenothiazine, phenoxazine, phthalazine, naphthyridine, quinazoline, cinnoline, carbazole, β-carboline, acridine, phenazine, phthalimide and the like can be mentioned.

The "aromatic ring" is preferably a benzene ring, a condensed aromatic hydrocarbon ring (preferably naphthalene etc.) having 9 to 14 carbon atoms, a 5- or 6-membered aromatic heterocyclic ring (preferably pyridine, isoxazole etc.) and the like.

As the "substituents" of the "aromatic ring optionally further having 1 to 3 substituents" for ring A, an "aliphatic hydrocarbon group optionally having substituents" and the "halogen atom", "nitro group", "hydroxy group optionally having substituents", "amino group optionally having substituents", "acyl group" and the like, which have been recited as the examples of the aforementioned substituents of the "hydrocarbon group optionally having substituents" for R or R¹ can be mentioned.

Here, as the "aliphatic hydrocarbon group" of the "aliphatic hydrocarbon group optionally having substituents", those recited as the examples of the "hydrocarbon group" of the aforementioned "hydrocarbon group optionally having substituents" for R or R¹ can be mentioned. Of those, a C₁₋₁₀ alkyl group, a C₂₋₁₀ alkenyl group and a C₂₋₁₀ alkynyl group are preferable.

As the "substituents" of the "aliphatic hydrocarbon group optionally having substituents", for example, a C₃₋₁₀ cycloalkyl group; a C₆₋₁₄ aryl group (e.g., phenyl, naphthyl etc.); an aromatic heterocyclic group (e.g., thienyl, furyl, pyridyl, oxazolyl, thiazolyl etc.); a non-aromatic heterocyclic group (e.g., tetrahydrofuryl, morpholino, thiomorpholino, piperidino, pyrrolidinyl, piperazinyl etc.); a C₇₋₁₀ aralkyl group; an amino group optionally mono- or di-substituted by a C₁₋₆ alkyl group or a C₂₋₈ acyl group (e.g., an alkanoyl group etc.); an amidino group; a C₂₋₈ alkanoyl group; a carbamoyl group optionally mono- or di-substituted by a C₁₋₆ alkyl group; a sulfamoyl group optionally mono- or di-substituted by a C₁₋₆ alkyl group; a carboxyl group; a C₂₋₈ alkoxycarbonyl group; a hydroxy group; a C₁₋₆ alkoxy group optionally substituted by 1 to 3 halogen atoms (e.g., fluorine, chlorine, bromine, iodine etc.); a C₂₋₅ alkenyloxy group optionally substituted by 1 to 3 halogen atoms (e.g., fluorine, chlorine, bromine, iodine etc.); a C₃₋₇ cycloalkyloxy group; a C₇₋₁₀ aralkyloxy group; a C₆₋₁₄ aryloxy group (e.g., phenyloxy, naphthyloxy etc.); a thiol group; a C₁₋₆ alkylthio group optionally substituted by 1 to 3 halogen atoms (e.g., fluorine, chlorine, bromine, iodine etc.); a C₇₋₁₀ aralkylthio group; a C₆₋₁₄ arylthio group (e.g., phenylthio, naphthylthio etc.); a sulfo group; a cyano group; an azide group; a nitro group; a nitroso group; a halogen atom (e.g., fluorine, chlorine, bromine, iodine) and the like can be mentioned. The number of substituents is, for example, 1 to 3.

The "substituent" for ring A is preferably a C₁₋₆ alkyl group, a hydroxy group, a C₁₋₆ alkoxy group, a C₇₋₁₀ aralkyloxy group (preferably benzyloxy) or a halogen atom.

Ring A is preferably "a benzene ring or a pyridine ring each optionally further having 1 to 3 substituents", more preferably a benzene ring or a pyridine ring each optionally further having 1 to 3 substituents selected from a C₁₋₆ alkyl group, a hydroxy group, a C₁₋₆ alkoxy group, a C₇₋₁₀ aralkyloxy group and a halogen atom. Ring A is particularly preferably a benzene ring.

r is an integer of 1 to 8, preferably an integer of 1 to 3.

As the "leaving group" for T, for example, halogen atoms (e.g., chlorine, bromine, iodine etc.), optionally halogenated C₁₋₆ alkylsulfonyloxy (e.g., methanesulfonyloxy, ethanesulfonyloxy, trifluoromethanesulfonyloxy etc.), C₆₋₁₀ arylsulfonyloxy optionally having substituents, hydroxy and the like can be mentioned.

As the "substituents" of the "C₆₋₁₀ arylsulfonyloxy optionally having substituents", for example, halogen atoms (e.g., chlorine, bromine, iodine etc.), optionally halogenated C₁₋₆ alkyl, optionally halogenated C₁₋₆ alkoxy and the like can be mentioned. The number of substituents is, for example, 1 to 3. Specific examples of the "C₆₋₁₀ arylsulfonyloxy optionally having substituents" include benzenesulfonyloxy, p-toluenesulfonyloxy, 1-naphthalenesulfonyloxy, 2-naphthalenesulfonyloxy and the like.

The "leaving group" preferably includes halogen atoms (e.g., chlorine, bromine, iodine etc.), methanesulfonyloxy, trifluoromethanesulfonyloxy, p-toluenesulfonyloxy and the like.

As a preferable combination of R⁴, X, q, Y, ring A and r, the following combination can be mentioned.

R⁴ is a 5- or 6-membered aromatic heterocyclic group (preferably furyl, thienyl, pyridyl, pyrimidinyl, pyrazinyl, oxazolyl, thiazolyl, triazolyl, oxadiazolyl or pyrazolyl);
optionally having 1 or 2 substituents selected from
1) furyl, thienyl, pyridyl, pyrazinyl, phenyl or naphthyl, each optionally having 1 to 3 substituents selected from a C₁₋₆ alkyl group optionally substituted by 1 to 3 halogen atoms (e.g., fluorine, chlorine, bromine, iodine etc.), a C₁₋₆ alkoxy group optionally substituted by 1 to 3 halogen atoms (e.g., fluorine, chlorine, bromine, iodine etc.), a halogen atom (e.g., fluorine, chlorine, bromine, iodine etc.), a nitro group, a hydroxy group and an amino group; and
2) a C₁₋₆ alkyl group or a C₃₋₇ cycloalkyl group, each optionally having 1 to 3 substituents selected from a C₁₋₆ alkoxy group optionally having 1 to 3 halogen atoms (e.g., fluorine, chlorine, bromine, iodine etc.), a halogen atom (e.g., fluorine, chlorine, bromine, iodine etc.), a nitro group, a hydroxy group and an amino group; and
optionally condensed with a benzene ring;
X is a bond or -NR⁷- (R⁷ is a hydrogen atom or a C₁₋₆ alkyl group) ;
q is 1 or 2;
Y is an oxygen atom, a sulfur atom, -NH- or -NHCO-;
ring A is a benzene ring, a condensed aromatic hydrocarbon ring (preferably naphthalene etc.) having 9 to 14 carbon atoms or a 5- or 6-membered aromatic heterocyclic ring (preferably pyridine, isoxazole etc.), each optionally further having 1 to 3 substituents selected from a C₁₋₆ alkyl group, a hydroxy group, a C₁₋₆ alkoxy group, a C₇₋₁₀ aralkyloxy group and a halogen atom; and
r is an integer of 1 to 3.
R⁰ is a hydrogen atom or a hydrocarbon group optionally having substituents. Here, as the "hydrocarbon group optionally having substituents", those same as the aforementioned "hydrocarbon group optionally having substituents" recited as the example of R¹ can be mentioned. R⁰ is preferably a hydrogen atom.

With regard to compound (II) used for the production method of the present invention, a compound wherein Q is a hydroxy group is equal to a compound represented by the formula 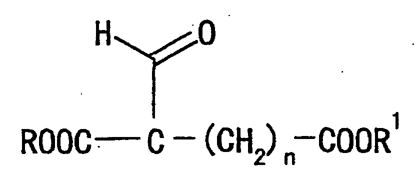 wherein the symbols in the formula are as defined above.

The production methods of the present invention are described in detail in the following.

When compounds (I) , (II) , (III) , (IV) , (V), (VI) , (VII) and (VIII) [hereinafter these compounds are sometimes to be abbreviated as the compounds of the present invention] used for the production method of the present invention are salts, such salts include, for example, salts with inorganic bases, salts with organic bases, salts with inorganic acids, salts with organic acids, salts with basic or acidic amino acids and the like.

Preferable examples of the salts with inorganic bases include alkali metal salts such as sodium salts, potassium salts etc.; alkaline earth metal salts such as calcium salts, magnesium salts etc.; aluminum salts, ammonium salts and the like.

Preferable examples of the salts with organic bases include salts with trimethylamine, triethylamine, pyridine, picoline, ethanolamine, diethanolamine, triethanolamine, dicyclohexylamine, N,N-dibenzylethylenediamine and the like.

Preferable examples of the salts with inorganic acids include salts with hydrochloric acid, hydrobromic acid, nitric acid, sulfuric acid, phosphoric acid and the like.

Preferable examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acids include salts with arginine, lysine, ornithine and the like.

Preferable examples of the salts with acidic amino acids include salts with aspartic acid, glutamic acid and the like.

The compound of the present invention may be labeled with an isotope (e.g., ³H, ¹⁴C, ³⁵S, ¹²⁵I etc.) and the like, and may be an anhydride or a hydrate.

The compound (I) can be produced by reacting compound (II) with hydrazine or a salt thereof.

Here, as the salt of hydrazine, for example, a salt with an inorganic acid, a salt with an organic acid, a salt with an acidic amino acid and the like can be mentioned. As these salts, those similar to the salts mentioned above can be used. The salt of hydrazine is preferably a salt with an inorganic acid, more preferably hydrochloride.

The hydrazine or a salt thereof may be an anhydride or a hydrate.

The amount of the hydrazine or a salt thereof to be used is generally 1 to 10 molar equivalents, preferably 1 to 3 molar equivalents, relative to compound (II).

This reaction is generally carried out in a solvent that does not adversely influence the reaction. Examples of such solvent include aromatic hydrocarbons such as benzene, toluene, xylene etc.; ethers such as tetrahydrofuran, dioxane, diethyl ether, diisopropyl ether, tert-butyl methyl ether etc.; halogenated hydrocarbons such as chloroform, dichloromethane etc.; amides such as N,N-dimethylformamide etc.; sulfoxides such as dimethyl sulfoxide etc.; alcohols such as methanol, ethanol, isopropanol etc.; esters such as ethyl acetate etc.; water; and the like. These solvents may be used in mixture at appropriate ratios.

The reaction temperature is generally -50 to 150°C, preferably -10 to 100°C.

The reaction time is generally 0.5-24 hrs., preferably 1-10 hrs.

When compound (II) is, for example, a salt such as an alkali metal salt and the like, the salt is preferably neutralized with a weak acid such as acetic acid and the like and then reacted with hydrazine or a salt thereof.

The compound (I) thus obtained can be isolated or purified by known separation and purification means, such as concentration, concentration under reduced pressure, solvent extraction, 'crystallization, recrystallization, phase transfer, chromatography and the like. In addition, a reaction mixture containing compound (I) may be subjected to the next reaction as it is without isolation and purification of the compound.

The above-mentioned compound (II) can be produced according to a method known *per se*. Also, a reaction mixture containing compound (II) thus produced may be subjected to a reaction as a starting material, without isolation and purification of the compound. In addition, hydrazine and a salt thereof are commercially available.

The compound (III) can be produced by subjecting compound (I) to an acylation reaction.

The acylation reaction is carried out according to a method known *per se*, such as a method comprising direct condensation of compound (I) with a compound represented by the formula (IX): Z-OH (Z is an acyl group) [hereinafter sometimes to be abbreviated as compound (IX)] using a condensing agent (e.g., dicyclohexylcarbodiimide etc.), or a method comprising appropriate reaction of a reactive derivative of compound (IX) with compound (I), and the like. Here, as the reactive derivative of compound (IX), for example, acid halides (e.g., acid chloride, acid bromide), acid anhydrides, imidazolide, or mixed acid anhydrides (e.g., anhydride with methyl carbonic acid, ethyl carbonic acid, isobutyl carbonic acid etc.) and the like can be mentioned.

When acid halide is used as a reactive derivative of compound (IX), for example, the reaction is carried out in the presence of a base in a solvent that does not adversely influence the reaction.

As the base, for example, amines such as triethylamine, N-methylmorpholine, N,N-dimethylaniline etc.; alkali metal carbonates such as sodium hydrogen carbonate, sodium carbonate, potassium carbonate etc.; alkali metal hydroxides such as sodium hydroxide, potassium hydroxide etc.; and the like can be mentioned.

The amount of the base to be used is generally 0.1 to 10 molar equivalents, preferably 1 to 3 molar equivalents, relative to compound (IX).

As the solvent that does not adversely influence the reaction, for example, aromatic hydrocarbons such as benzene, toluene, xylene etc.; ethers such as tetrahydrofuran, dioxane, diethyl ether, diisopropyl ether, tert-butyl methyl ether etc.; halogenated hydrocarbons such as chloroform, dichloromethane etc.; amides such as N,N-dimethylformamide etc.; sulfoxides such as dimethyl sulfoxide etc.; alcohols such as methanol, ethanol, isopropanol etc.; esters such as ethyl acetate etc.; water; and the like can be mentioned. These solvents may be used in mixture at appropriate ratios.

The amount of the compound (I) to be used is generally 0.1 to 10 molar equivalents, preferably 0.3 to 3 molar equivalents, relative to compound (IX).

The reaction temperature is generally -50 to 150°C, preferably -10 to 100°C.

The reaction time is generally 0.5-24 hrs., preferably 0.5-5 hrs.

In addition, when a mixed acid anhydride is used as a reactive derivative of compound (IX), for example, compound' (IX) is reacted with a chlorocarbonic acid ester in the presence of a base and then reacted with compound (I).

As the chlorocarbonic acid ester, for example, methyl chlorocarbonate, ethyl chlorocarbonate, isobutyl chlorocarbonate and the like can be mentioned.

The amount of the chlorocarbonic acid ester to be used is generally 0.1 to 10 molar equivalents, preferably 0.3 to 3 molar equivalents, relative to compound (IX).

As the base, for example, amines such as triethylamine, N-methylmorpholine, N,N-dimethylaniline etc.; alkali metal carbonates such as sodium hydrogen carbonate, sodium carbonate, potassium carbonate etc.; and the like can be mentioned.

The amount of the base to be used is generally 0.1 to 10 molar equivalents, preferably 0.3 to 3 molar equivalents, relative to compound (IX).

The amount of the compound (I) to be used is generally 0.1 to 10 molar equivalents, preferably 0.3 to 3 molar equivalents, relative to compound (IX).

The reaction temperature is generally -50 to 150°C, preferably -10 to 100°C.

The reaction time is generally 0.5-24 hrs., preferably 0.5-5 hrs.

The compound (III) thus obtained can be isolated or purified by known separation and purification means, such as concentration, concentration under reduced pressure, solvent extraction, crystallization, recrystallization, phase transfer, chromatography and the like. In addition, a reaction mixture containing compound (III) may be subjected to the next reaction as it is without isolation and purification of the compound.

For example, the above-mentioned compound (IX) is commercially available.

The compound (IV) can be produced by subjecting compound (III) to an alkylation reaction.

The alkylation reaction is carried out according to a method known per se using an alkylating agent.

As the alkylating agent, a compound represented by the formula: R³-T (the symbols in the formula are as defined above) can be mentioned.

Preferable examples of the alkylating agent include methyl chloride, methyl bromide, methyl iodide, ethyl chloride, ethyl bromide, ethyl iodide, 1-propyl chloride, 1-propyl bromide, 1-propyl iodide, 2-propyl chloride, 2-propyl bromide, 2-propyl iodide, dimethyl sulfate, diethyl sulfate and the like.

The amount of the alkylating agent to be used is generally 0.5 to 10 molar equivalents, preferably 1 to 3 molar equivalents, relative to compound (III).

This reaction is carried out in, for example, a solvent that does not adversely influence the reaction in the presence of a base.

As the base, for example, amines such as triethylamine, N-methylmorpholine, N,N-dimethylaniline etc.; alkali metal carbonates such as sodium hydrogen carbonate, sodium carbonate, potassium carbonate etc.; alkali metal alkoxides such as sodium methoxide, sodium ethoxide, sodium tert-butoxide, potassium tert-butoxide etc.; alkali metal hydrides such as potassium hydride, sodium hydride etc.; and the like can be mentioned.

The amount of the base to be used is generally 0.5 to 10 molar equivalents, preferably 1 to 3 molar equivalents, relative to compound (III).

As the solvent that does not adversely influence the reaction, for example, aromatic hydrocarbons such as benzene, toluene, xylene etc.; ethers such as tetrahydrofuran, dioxane, diethyl ether, diisopropyl ether, tert-butyl methyl ether etc.; halogenated hydrocarbons such as chloroform, dichloromethane etc.; amides such as N,N-dimethylformamide etc.; sulfoxides such as dimethyl sulfoxide etc.; alcohols such as methanol, ethanol, isopropanol etc.; esters such as ethyl acetate etc.; water; and the like can be mentioned. These solvents may be used in mixture at appropriate ratios.

The reaction temperature is generally -50 to 150°C, preferably -10 to 100°C.

The reaction time is generally 0.5-24 hrs., preferably 1-10 hrs.

The compound (IV) thus obtained can be isolated or purified by known separation and purification means, such as concentration, concentration under reduced pressure, solvent extraction, crystallization, recrystallization, phase transfer, chromatography and the like. In addition, a reaction mixture containing compound (IV) may be subjected to the next reaction as it is without isolation and purification of the compound.

For example, the above-mentioned alkylating agent is commercially available.

The compound (V) can be produced by subjecting compound (IV) to a deacylation reaction.

The deacylation reaction can be carried out according to a method known *per se*. The deacylation reaction is carried out by, for example, maintaining compound (IV) in an acidic or basic aqueous solution at generally -20°C to 200°C, preferably 20°C to 140°C.

As the acid, for example, a hydrochloric acid, a sulfuric acid, a hydrobromic acid, an iodic acid, a periodic acid and the like can be mentioned. As the base, for example, alkali metal hydroxides such as sodium hydroxide, potassium hydroxide, lithium hydroxide etc.; and the like can be mentioned.

The amount of the acid or base to be used is generally 1 to 100 equivalents, preferably 1 to 40 equivalents, relative to compound (IV). The strength of the acid or base is generally 0.1N to 18N, preferably 1N to 12N.

The reaction time is generally 0.5-48 hrs., preferably 1-24 hrs.

When Z is a tert-butoxycarbonyl group and the like, the deacylation reaction is also carried out by dissolving compound (IV) in an organic acid (e.g., trifluoroacetic acid, formic acid, acetic acid, methanesulfonic acid, benzenesulfonic acid, trifluoromethanesulfonic acid etc.) and maintaining the solution at generally -20°C to 200°C, preferably 0°C to 100°C. The amount of the organic acid to be used is generally 1 to 100 equivalents, preferably 1 to 40 equivalents, relative to compound (IV).

Moreover, the deacylation reaction is also carried out by subjecting compound (IV) to a catalytic reduction using palladium, palladium-carbon, Raney-nickel, Raney-cobalt, platinum oxide and the like as a catalyst in a solvent such as alcohol solvents (e.g., ethanol etc.), acetic acid and the like at atmospheric pressure or under pressurization as necessary.

The compound (V) thus obtained can be isolated or purified by known separation and purification means, such as concentration, concentration under reduced pressure, solvent extraction, crystallization, recrystallization, phase transfer, chromatography and the like. In addition, a reaction mixture containing compound (V) may be subjected to the next reaction as it is without isolation and purification of the compound.

The compound (VII) [R⁰ = a hydrocarbon group optionally having substituents] can be produced by reacting compound (V) with compound (VI).

When, in compound (VI), T is a halogen atom, optionally halogenated C₁₋₆ alkylsulfonyloxy or C₆₋₁₀ arylsulfonyloxy optionally having substituents, this reaction is carried out in the presence of a base in a solvent that does not adversely influence the reaction.

As the base, for example, alkali metal hydroxides such as potassium hydroxide, sodium hydroxide etc.; alkali metal carbonates such as sodium hydrogen carbonate, potassium carbonate etc.; amines such as pyridine, triethylamine, N,N-dimethylaniline, 1,8-diazabicyclo[5.4.0]undec-7-ene etc.; alkali metal hydrides such as potassium hydride, sodium hydride etc.; alkali metal alkoxides such as sodium methoxide, sodium ethoxide, sodium tert-butoxide, potassium tert-butoxide etc.; can be mentioned.

The amount of the base to be used is generally 0.5 to 10 molar equivalents, preferably 1 to 5 molar equivalents, relative to compound (V).

As the solvent that does not adversely influence the reaction, for example, aromatic hydrocarbons such as benzene, toluene, xylene etc.; ethers such as tetrahydrofuran, dioxane, diethyl ether, diisopropyl ether, tert-butyl methyl ether etc.; ketones such as acetone, 2-butanone etc.; halogenated hydrocarbons such as chloroform, dichloromethane etc.; amides such as N,N-dimethylformamide etc.; sulfoxides such as dimethyl sulfoxide etc.; and the like can be mentioned. These solvents may be used in mixture at appropriate ratios.

The amount of the compound (VI) to be used is generally 0.5 to 10 molar equivalents, preferably 1 to 5 molar equivalents, relative to compound (V).

The reaction temperature is generally -50 to 150°C, preferably -10 to 100°C.

The reaction time is generally 0.5-20 hrs., preferably 1-10 hrs.

When, in compound (VI), T is hydroxy, moreover, this reaction is carried out according to a method known *per se,* such as the method described in, for example, Synthesis, page 1 (1981), or a method analogous thereto. That is, this reaction is carried out in the presence of an organic phosphorus compound and an electrophile in a solvent that does not adversely influence the reaction.

As the organic phosphorus compound, for example, triphenylphosphine, tributylphosphine and the like can be mentioned.

As the electrophile, for example, diethyl azodicarboxylate, diisopropyl azodicarboxylate, azodicarbonyl dipiperazine and the like can be mentioned.

The amount of the organic phosphorus compound and the electrophile to be used is generally 0.5 to 10 molar equivalents, preferably 1 to 5 molar equivalents, relative to compound (V).

As the solvent that does not adversely influence the reaction, for example, ethers such as tetrahydrofuran, dioxane, diethyl ether, diisopropyl ether, tert-butyl methyl ether etc.; halogenated hydrocarbons such as chloroform, dichloromethane etc.; aromatic hydrocarbons such as benzene, toluene, xylene etc.; amides such as N,N-dimethylformamide etc.; sulfoxides such as dimethyl sulfoxide etc.; and the like can be mentioned. These solvents may be used in mixture at appropriate ratios.

The amount of the compound (VI) to be used is generally 0.5 to 10 molar equivalents, preferably 1 to 5 molar equivalents, relative to compound (V).

The reaction temperature is generally -50 to 150°C, preferably -10 to 100°C.

The reaction time is generally 0.5-20 hrs., preferably 1-10 hrs.

The compound (VII) [R⁰ = a hydrocarbon group optionally having substituents] thus obtained can be isolated or purified by known separation and purification means, such as concentration, concentration under reduced pressure, solvent extraction, crystallization, recrystallization, phase transfer, chromatography and the like. In addition, a reaction mixture containing compound (VII) [R⁰ = a hydrocarbon group optionally having substituents] may be subjected to the next reaction as it is without isolation and purification of the compound.

The above-mentioned compound (VI) can be produced according to a method known *per se*, such as the method described in, for example, WO 01/38325 and the like or a method analogous thereto.

The compound (VII) [R⁰ = a hydrogen atom] can be produced by subjecting compound (VII) [R⁰ = a hydrocarbon group optionally having substituents] to a hydrolysis reaction.

The hydrolysis reaction is carried out according to conventional methods in the presence of an acid or a base in water-containing solvent.

As the acid, for example, a hydrochloric acid, a sulfuric acid, an acetic acid, a hydrobromic acid and the like can be mentioned.

As the base, for example, alkali metal carbonates such as potassium carbonate, sodium carbonate etc.; alkali metal alkoxides such as sodium methoxide etc.; alkali metal hydroxides such as potassium hydroxide, sodium hydroxide, lithium hydroxide etc.; and the like can be mentioned.

The amount of the acid or base to be used is generally an excess amount of compound (VII) [R⁰ = a hydrocarbon group optionally having substituents]. Preferably, the amount of the acid to be used is 2 to 50 molar equivalents relative to compound (VII) [R⁰= a hydrocarbon group optionally having substituents], and the amount of the base to be used is 1.2 to 5 molar equivalents relative to compound (VII) [R⁰ = a hydrocarbon group optionally having substituents].

As the water-containing solvent, for example, a mixed solvent of one or more solvents selected from alcohols such as methanol, ethanol etc.; ethers such as tetrahydrofuran, dioxane, diethyl ether, diisopropyl ether, tert-butyl methyl ether etc.; amides such as N,N-dimethylformamide etc.; sulfoxides such as dimethyl sulfoxide etc.; and ketones such as acetone etc. with water and the like can be mentioned.

The reaction temperature is generally -20 to 150°C, preferably -10 to 100°C.

The reaction time is generally 0.1-20 hrs., preferably 1-5 hrs.

The compound (VII) [R⁰ = a hydrogen atom] thus obtained can be isolated or purified by known separation and purification means, such as concentration, concentration under reduced pressure, solvent extraction, crystallization, recrystallization, phase transfer, chromatography and the like.

In addition, compound (VIII) can be produced by reacting compound (I) with compound (VI).

This reaction is carried out in the same manner as in the aforementioned reaction of compound (V) with compound (VI).

The compound (VIII) thus obtained can be isolated or purified by known separation and purification means, such as concentration, concentration under reduced pressure, solvent extraction, crystallization, recrystallization, phase transfer, chromatography and the like. In addition, a reaction mixture containing compound (VIII) may be subjected to the next reaction as it is without isolation and purification of the compound.

The compound (VII) [R⁰ = a hydrocarbon group optionally having substituents] can be produced by subjecting compound. (VIII) to an alkylation reaction.

This reaction is carried out in the same manner as in the aforementioned alkylation reaction of compound (III).

The compound (VII) [R⁰ = a hydrocarbon group optionally having substituents] thus obtained can be isolated or purified by known separation and purification means, such as concentration, concentration under reduced pressure, solvent extraction, crystallization, recrystallization, phase transfer, chromatography and the like. In addition, a reaction mixture containing compound (VII) [R⁰ = a hydrocarbon group optionally having substituents] may be subjected to the next reaction as it is without isolation and purification of the compound.

The compound (VII) and compound (VIII) obtained by the production method of the present invention have a hypoglycemic action, a hypolipidemic action, a hypoinsulinemic action, an insulin resistance improving action, an insulin sensitivity enhancing action and retinoid-related receptors [e.g., retinoid X receptors (RXRα, RXRβ, RXRγ), peroxisome proliferator activated receptors (PPARα, PPARβ (PPARδ), PPARγ) etc.] function regulating activity, and are useful as an agent for the prophylaxis or treatment of diabetes (e.g., type I diabetes, type II diabetes, gestational diabetes etc.); an agent for the prophylaxis or treatment of hyperlipidemia (e.g., hypertriglyceridemia, hypercholesterolemia, hypo-HDL-emia, postprandial hyperlipidemia etc.); an insulin sensitizer; an insulin sensitivity enhancer; an agent for the prophylaxis or treatment of impaired glucose tolerance (IGT); an agent for the prophylaxis or treatment of syndrome X; an agent for the prophylaxis or treatment of dysmetabolic syndrome and the like.

The compound (VII) and compound (VIII) can be formulated into a preparation according to the method described in WO 01/38325 and the like or a method analogous thereto, and administered to mammals.

The compound (VII) and compound (VIII) can be used in combination with a pharmaceutical agent (hereinafter to be abbreviated as a concomitant pharmaceutical agent) such as a therapeutic agent for diabetes, a therapeutic agent for diabetic complications, an antiepileptic agent, an antidepressant, an opioid agonist, an antihyperlipidemic agent, a hypotensive agent, an antiarrhythmic agent, an antiobesity agent, a diuretic, a chemotherapeutic agent, an immunotherapeutic agent, an antithrombotic agent, a therapeutic agent for osteoporosis, an antidementia agent, an agent for ameliorating erectile dysfunction, a therapeutic agent for incontinentia or pollakiuria, a non-steroidal anti-inflammatory drug, a local anesthetic, vitamins and the like. These concomitant pharmaceutical agents may be a low molecular weight compound, or may be a high molecular weight protein, polypeptide, antibody, vaccine and the like.

Examples of the therapeutic agent for diabetes include insulin preparations (e.g. animal insulin preparations obtained by extraction from the bovine or swine pancreas; human insulin preparations synthesized by a genetic engineering technique using *Escherichia coli* or a yeast; insulin-zinc; protamine-insulin-zinc; a fragment or derivative of insulin (e.g., INS-1 etc.)), insulin sensitizers (e.g. pioglitazone or its salt (preferably hydrochloride) , rosiglitazone or its salt (preferably maleate), reglixane (JTT-501), GI-262570, netoglitazone (MCC-555), YM-440, DRF-2593, BM-13.1258, KRP-297, R-119702, CS-011, FK-614, the compounds described in WO99/58510 (e.g., (E)-4-[4-(5-methyl-2-phenyl-4-oxazolylmethoxy)benzyloxyimino]-4-phenylbutyric acid), tesaglitazar (AZ-242), ragaglitazar (NN-622), BMS-298585, ONO-5816, BM-13-1258, LM-4156, MBX-102, LY-519818, MX-6054, LY-510929, and the like), α-glucosidase inhibitors (e.g., voglibose, acarbose, miglitol, emiglitate etc.), biguanides (e.g., phenformin, metformin, buformin etc.), insulin secretagogues [sulfonylureas (e.g., tolbutamide, glibenclamide, gliclazide, chlorpropamide, tolazamide, acetohexamide, glyclopyramide, glimepiride, glipizide, glybuzole etc.), repaglinide, senaglinide, nateglinide, mitiglinide or its calcium salt hydrate etc.], GLP-1 receptor agonists [e.g., GLP-1, NN-2211, AC-2993 (exendin-4), BIM-51077, Aib(8,35)hGLP-1(7,37)NH₂ etc.], amyrin agonists (e.g., pramlintide etc.), phosphotyrosine phosphatase inhibitors (e.g., vanadic acid etc.), dipeptidylpeptidase IV inhibitors (e.g., NVP-DPP-278, PT-100, P32/98, NVP-DDP-728, LAF237 etc.), β3 agonists (e.g., CL-316243, SR-58611-A, UL-TG-307, SB-226552, AJ-9677, BMS-196085, AZ40140 etc.), gluconeogenesis inhibitors (e.g., glycogen phosphorylase inhibitors, glucose-6-phosphatse inhibitors, glucagon antagonists etc.), SGLT (sodium-glucose cotransporter) inhibitors (e.g., T-1095 etc.) and the like.

Examples of the therapeutic agent for diabetic complications include aldose reductase inhibitors (e.g., tolrestat, epalrestat, zenarestat, zopolrestat, minalrestat, fidarestat, SNK-860, CT-112 etc.), neurotrophic factors and enhancers thereof (e.g., NGF, NT-3, BDNF, neurotrophin production/secretion promoters described in WO01/14372 (e.g., 4-(4-chlorophenyl)-2-(2-methyl-1-imidazolyl)-5-[3-(2-methylphenoxy)propyl]oxazole etc.) and the like), neuranegenesis promoters (e.g., Y-128 etc.), PKC inhibitors (e.g., LY-333531 etc.), AGE inhibitors (e.g., ALT946, pimagedine, pyratoxathine, N-phenacylthiazolium bromide (ALT766), EXO-226 etc.), active oxygen scavengers (e.g. thioctic acid etc.), cerebral vasodilators (e.g., tiapuride, mexiletine etc.) and the like.

Examples of the antiepileptic agent include gabapentin, carbamazepine and the like.

Examples of the antidepressant include amitriptyline, imipramine and the like.

Examples of the opioid agonist include morphine and the like.

Examples of the antihyperlipidemic agent include statin compounds (e.g., cerivastatin, pravastatin, simvastatin, lovastatin, atorvastatin, fluvastatin, itavastatin or their salts (e.g., sodium salt) etc.), squalene synthase inhibitors (e.g., compounds described in WO97/10224 such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compounds (e.g., bezafibrate, clofibrate, simfibrate, clinofibrate etc.), antioxidants (e.g., lipoic acid, probucol) and the like.

Examples of the hypotensive agent include angiotensin converting enzyme inhibitors (e.g., captopril, enalapril, delapril etc.), angiotensin II antagonists (e.g., candesartan cilexetil, losartan, eprosartan, valsartan, termisartan, irbesartan, tasosartan etc.), calcium antagonists (e.g., manidipine, nifedipine, amlodipine, efonidipine, nicardipine etc.), clonidine and the like.

Examples of the antiarrhythmic agent include mexiletine and the like.

Examples of the antiobesity agent include antiobesity drugs acting on the central nervous system (e.g. dexfenfluramine, fenfluramine, phentermine, sibutramine, anfepramon, dexamphetamine, mazindol, phenylpropanolamine, clobenzorex; MCH receptor antagonists (e.g., SB-568849; SNAP-7941; compounds included in WO01/82925 and WO01/87834 etc.); neuropeptide Y antagonists (e.g., CP-422935 etc.); cannabinoid receptor antagonists (e.g., SR-141716, SR-147778 etc.); ghrelin antagonists; 11β-hydroxysteroid dehydrogenase inhibitors (e.g., BVT-3498 etc.) and the like), pancreatic lipase inhibitors (e.g. orlistat, ALT-962 etc.), β3 agonists (e.g. CL-316243, SR-58611-A, UL-TG-307, SB-226552, AJ-9677, BMS-196085, AZ40140 etc.); anorectic peptides (e.g. leptin, CNTF(Ciliary Neurotrophic Factor) etc.), cholecystokinin agonists (e.g. lintitript, FPL-15849 etc.) and the like.

Examples of the diuretic include xanthine derivatives (e.g., theobromine and sodium salicylate, theobromine and calcium salicylate etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide etc.), antialdosterone preparations (e.g., spironolactone, triamterene etc.), carbonate dehydratase inhibitors (e.g., acetazolamide etc.), chlorobenzenesulfonamide preparations (e.g., chlorthalidone, mefruside, indapamide etc.), azosemide, isosorbide, ethacrynic acid, piretanide, bumetanide, furosemide and the like.

Examples of the chemotherapeutic agent include alkylating agents (e.g., cyclophosphamide, ifosfamide etc.), metabolic antagonists (e.g., methotrexate, 5-fluorouracil etc.), antitumor antibiotics (e.g., mitomycin, adriamycin etc.), plant-derived antitumor agents (e.g., vincristine, vindesine, Taxol etc.), cisplatin, carboplatin, etoposide and the like. Of these, 5-fluorouracil derivatives such as Furtulon and Neo-Furtulon and the like are preferable.

Examples of the immunotherapeutic agent include microorganism- or bacterium-derived components (e.g., muramyl dipeptide derivatives, Picibanil etc.), immunopotentiator polysaccharides (e.g., lentinan, schizophyllan, krestin etc.), genetically engineered cytokines (e.g., interferons, interleukins (IL) etc.), colony stimulating factors (e.g., granulocyte colony stimulating factor, erythropoietin etc.), and the like. Of these, interleukins such as IL-1, IL-2, IL-12 and the like are preferable.

Examples of the antithrombotic agents include heparins (e.g., heparin sodium, heparin calcium, dalteparin sodium etc.), warfarins (e.g., warfarin potassium etc.), antithrombin agents (e.g., aragatroban etc.), thrombolytic agents (e.g., urokinase, tisokinase, alteplase, nateplase, monteplase, pamiteplase etc.), platelet aggregation suppressants (e.g., ticlopidine hydrochloride, cilostazol, ethyl icosapentaenoate, beraprost sodium, sarpogrelate hydrochloride etc.) and the like.

Examples of the therapeutic agent for osteoporosis include alfacalcidol, calcitriol, elcaltonin, calcitonin salmon, estriol, ipriflavone, pamidronate disodium, alendronate sodium hydrate, incadronate disodium and the like.

Examples of the antidementia agent include tacrine, donepezil, rivastigmine, galantamine and the like.

Examples of the agent for ameliorating erectile dysfunction include apomorphine, sildenafil citrate and the like.

Examples of the therapeutic agent for incontinentia or pollakiuria include flavoxate hydrochloride, oxybutynin hydrochloride, propiverine hydrochloride and the like.

Examples of the non-steroidal anti-inflammatory drug include aspirin, acetaminophen, indomethacin and the like.

Examples of the local anesthetic include lidocaine, capsaicin and the like.

Examples of vitamins include vitamin B1, vitamin B12 and the like.

The timing of administration of the aforementioned concomitant pharmaceutical agent is not limited. The compound (VII) or compound (VIII) and a concomitant pharmaceutical agent may be administered simultaneously or at staggered times to the administration subject. The dose of the concomitant pharmaceutical agent can be appropriately determined based on the dose clinically employed, and it can be appropriately selected according to the administration subject, administration route, disease, combination, and other factors.

The administration mode of the concomitant pharmaceutical agent is not particularly limited, as long as compound (VII) or compound (VIII) and the concomitant pharmaceutical agent are combined on administration. Examples of such administration mode include the following methods: (1) The compound (VII) or compound (VIII) and the concomitant pharmaceutical agent are simultaneously processed to give a single preparation which is administered. (2) The compound (VII) or compound (VIII) and the concomitant pharmaceutical agent are separately processed to give two kinds of preparations which are administered simultaneously by the same administration route. (3) The compound (VII) or compound (VIII) and the concomitant pharmaceutical agent are separately processed to give two kinds of preparations which are administered by the same administration route at staggered times. (4) The compound (VII) or compound (VIII) and the concomitant pharmaceutical agent are separately processed to give two kinds of preparations which are administered simultaneously by the different administration routes. (5) The compound (VII) or compound (VIII) and the concomitant pharmaceutical agent are separately processed to give two kinds of preparations which are administered by the different administration routes at staggered times (for example, compound (VII) or compound (VIII) and the concomitant pharmaceutical agent are administered in this order, or in the reverse order), and the like.

The proportion of compound (VII) or compound (VIII) to the concomitant pharmaceutical agent can be appropriately selected depending on an administration subject, administration route, diseases and the like. For example, when the administration subject is a human, 0.01-100 parts by weight of the concomitant pharmaceutical agent may be used relative to 1 part by weight of compound (VII) or compound (VIII).

When compound (VII) or compound (VIII) is used in combination with a concomitant pharmaceutical agent, the dose of the both components can be reduced within a safe range in consideration of the opposing effect of the components. Particularly, concomitant pharmaceutical agents such as an insulin sensitizer, an insulin secretagogue (preferably a sulfonylurea), a biguanide, an aldose reductase inhibitor, a PKC inhibitor, an antiepileptic agent, an antidepressant, an antiarrhythmic agent, an opioid agonist, an antioxidant, a non-steroidal anti-inflammatory drug and the like can be reduced from the normal dose. Therefore, the opposing effect caused by these pharmaceutical agents can be safely prevented.

The present invention is explained in detail by referring to Reference Examples, Examples and Formulation Examples, which are not to be construed as limitative. In the following Reference Examples and Examples, % means percent by weight unless specifically indicated. In addition, room temperature means a temperature of 1-30°C.

Moreover, magnesium stearate used in Formulation Examples was a product meeting the standards of the Japan Pharmacopoeia 14th Ed.

### Examples

### Reference Example 1

### {4-[(2-Phenyl-1,3-thiazol-4-yl)methoxy]phenyl}methanol

A mixture of thiobenzamide (5.488 g), 1,3-dichloroacetone (5.079 g) and toluene (40 mL) was stirred at 90°C for 3 hrs. To this reaction mixture was added isopropanol (40 mL) and the mixture was stirred at 80°C for 30 min. The mixture was allowed to cool to give 4-chloromethyl-2-phenylthiazole. To the reaction mixture were added potassium carbonate (13.82 g), p-hydroxybenzaldehyde (4.885 g) and dimethylacetamide (40 mL) and the mixture was stirred at 90°C for 1 hr. to give 4-[(2-phenyl-1,3-thiazol-4-yl)methoxy]benzaldehyde. A part of the reaction mixture was taken, crystallized by conventional treatments and used for measurement of NMR.
¹H-NMR(300MHz, CDCl₃) δ: 5.36(2H, s), 7.13-7.48(6H, m) , 7.85-7.98 (4H, m), 9.91(1H, s).

The remaining reaction mixture was cooled under an N₂ atmosphere and sodium borohydride (1.589 g) was added at 5°C. The mixture was stirred at 20-30°C for 1 hr. The internal temperature was set to 40°C and methanol (6 mL) and water (70 mL) were added. The mixture was stirred at room temperature. The precipitated crystals were collected by filtration, washed with 50% aqueous methanol solution and then with water to give the title compound.
¹H-NMR(300MHz, CDCl₃) δ: 1.61 (1H,bs),4.63(2H, s), 5.27(2H, s) , 7.00(2H, m), 7.29-7.32 (3H, m), 7.43-7.48(3H, m), 7.93-7.97(2H, m).

### Reference Example 2

### {4-[(2-Phenyl-1,3-thiazol-4-yl)methoxy]phenyl}methanol

Dichloroacetone (5.55 g) was dissolved in toluene (60 mL) and thiobenzamide (6 g) was added. The mixture was stirred at about 90°C for 3 hrs. 2-Propanol (45 mL) was added to the reaction mixture and the mixture was stirred under reflux for 30 min. The reaction mixture was cooled to near room temperature and washed with 10% brine. The organic layer was concentrated to give 4-chloromethyl-2-phenylthiazole.
¹H-NMR(300MHz, CDCl₃) δ: 4.75(2H, s), 7.31(1H, s), 7.40-7.50(3H, m), 7.90-8:00(2H, m).

N,N-Dimethylacetamide (48 mL) and p-hydroxybenzyl alcohol (5.43 g) were added to the concentrated solution and 28% sodium methoxide-methanol solution (8.44 g) was added dropwise at room temperature. The mixture was stirred at about 70°C for 1.5 hrs. The reaction mixture was cooled to room temperature and water (60 mL) was added dropwise. The precipitated crystals were collected by filtration and washed successively with 50% aqueous methanol, water and ethyl acetate. The obtained crystals (10.85 g) were dissolved in ethyl acetate (217 mL) at 70°C and activated carbon (0.54 g) was added. The mixture was stirred at 70°C. Activated carbon was filtered off while the mixture was hot, and ethyl acetate was evaporated to an about half amount. n-Heptane (108 ml) was added dropwise to the residue at room temperature and the precipitated crystals were collected by filtration to give the title compound as pale-yellow crystals.
¹H-NMR(300MHz, CDCl₃) δ:1.61(1H,bs),4.63(2H, s), 5.27(2H, s), 7.00(2H, m), 7.29-7.32(3H, m), 7.43-7.48(3H, m), 7.93-7.97(2H, m).

### Reference Example 3

### 4-{[4-(Chloromethyl)phenoxy]methyl}-2-phenyl-1,3-thiazole

{4-[(2-Phenyl-1,3-thiazol-4-yl)methoxy]phenyl}methanol (4.35 g) was added to N,N-dimethylformamide (13 mL) and thionyl chloride (0.87 mL) was added dropwise at not more than 20°C. The mixture was stirred at room temperature for 1 hr. Ethyl acetate (52 mL) and water (13 mL) were added to the reaction mixture for extraction. The organic layer was washed with 10% aqueous sodium carbonate solution and 10% brine and ethyl acetate was evaporated to give the title compound as yellow white crystals.
¹H-NMR(300MHz, CDCl₃) δ:4.58(2H, s) , 5.27(2H, s), 7.01(2H, d, J=6.7Hz), 7.30-7.47 (6H, m), 7.96 (2H, m).

### Example 1

### Ethyl 3-(3-hydroxy-1H-pyrazol-4-yl)propionate

Under ice-cooling, to a suspension of sodium tert-butoxide (24.5 g) in tert-butyl methyl ether (300 mL) was added dropwise a mixture of diethyl glutarate (47.05 g) and ethyl formate (24.2 mL) at not more than 10°C. The mixture was stirred at room temperature for 24 hrs. to give diethyl 2-formylglutarate. To the reaction mixture were added dropwise acetic acid (29 mL) and hydrazine monohydrate (6.8 mL) at room temperature, and the mixture was stirred under reflux for 2 hrs. Under reflux, water (120 mL) was added dropwise to the reaction mixture and the mixture was allowed to cool to room temperature. The mixture was stirred under ice-cooling for 1 hr. The precipitated crystals were collected by filtration and washed with water and diisopropyl ether to give the title compound.
¹H-NMR(300MHz, CDCl₃)δ: 1.03 (3H, t, J=7.1Hz), 2.32-2.37 (2H, m),2.45-2.50(2H, m), 3.91(2H, q, J=7.1Hz), 6.92(1H, s).

| Elemental analysis value: for C₈H₁₂N₂O₃ | | | |
|---|---|---|---|
| Calculated; | C, 52.17 | H, 6.57 | N, 15.21 |
| Found; | C, 52.08 | H, 6.49 | N, 15.16 |

### Example 2

### Ethyl 3-(3-hydroxy-1H-pyrazol-4-yl)propionate

To a mixture of diisopropyl ether (30 mL) and sodium ethoxide (1.74 g) was added dropwise a mixture of diethyl glutarate (4.71 g) and ethyl formate (2.22 g) at not more than 10°C and the mixture was stirred at room temperature for 24 hrs. to give diethyl 2-formylglutarate. Acetic acid (2.9 mL) and 50% aqueous hydrazine solution (3.1 mL) were added dropwise to the reaction mixture and the mixture was stirred with heating under reflux for 2 hrs. Water (12 mL) was added dropwise to the reaction mixture, and the mixture was cooled to room temperature. The precipitated crystals were collected by filtration, and washed with water and diisopropyl ether to give the title compound as a while crystals.
¹H-NMR(300MHz, DMSO-d₆) δ: 1.16(3H, m) , 2.42-2.72(4H, m), 4.05(2H, m), 7.15(1H, s), 9.5(1H, bs), 11.0(1H, bs).

### Example 3

### Benzyl 3-hydroxy-4-(3-ethoxy-3-oxopropyl)-1H-pyrazole-1-carboxylate

Ethyl 3-(3-hydroxy-1H-pyrazol-4-yl)propionate (10 g) and potassium carbonate (8.25 g) were added to N,N-dimethylformamide (40 mL), and benzyl chloroformate (8.5 mL) was added dropwise at not more than 15°C. The mixture was stirred at room temperature for 1 hr. Ethyl acetate (120 mL) and (80 mL) and tetrahydrofuran (60 mL) were added to the reaction mixture for extraction. The organic layer was washed with 10% brine and concentrated. n-Hexane was added to the residue and the precipitated crystals were collected by filtration to give the title compound.
¹H-NMR(300MHz, CDCl₃) δ:1.24(3H, m), 2.54(2H, m), 2.69(2H, m), 4.14(2H, m), 5.38(2H, s), 7.38-7.48(5H, m), 7.69(1H, s), 10.90(1H, bs).

### Example 4

### Benzyl 3-ethoxy-4-(3-ethoxy-3-oxopropyl)-1H-pyrazole-1-carboxylate

Benzyl 3-hydroxy-4-(3-ethoxy-3-oxopropyl)-1H-pyrazole-1-carboxylate (3.4 g), potassium carbonate (2.95 g) and ethyl iodide (1.11 mL) were added to N,N-dimethylformamide (13.6 mL) and the mixture was stirred at 50°C for 2 hrs. Ethyl acetate and dilute hydrochloric acid were added to the reaction mixture for extraction. The organic layer was washed with 10% brine and concentrated. The residue was purified by silica gel column chromatography to give the title compound as a colorless oil.
¹H-NMR(300MHz, CDCl₃) δ:1.21(3H, t), 1.39(3H, t), 2.55(2H, t), 2.67(2H, t), 4.12(2H, q), 4.35(2H, q), 5.39(2H, s), 7.34-7.46(5H, m), 7.71(1H, s).

### Example 5

### Benzyl 3-ethoxy-4-(3-ethoxy-3-oxopropyl)-1H-pyrazole-1-carboxylate

Ethyl 3-(3-hydroxy-1H-pyrazol-4-yl)propanoate (2 g) and potassium carbonate (1.6 g) were added to N,N-dimethylformamide (8 mL) and benzyl chloroformate (1.6 mL) was added dropwise at not more than 15°C and the mixture was stirred at room temperature for 1 hr. to give benzyl 3-hydroxy-4-(3-ethoxy-3-oxopropyl)-1H-pyrazole-1-carboxylate. Potassium carbonate (1.5 g) was added to the reaction mixture and diethyl sulfate (1.5 mL) was added dropwise at 50°C. The mixture was stirred at the same temperature for 2 hrs. Ethyl acetate and water were added to the reaction mixture for extraction. The organic layer was washed 3 times with 10% brine and ethyl acetate was evaporated. The residue was dissolved in ethanol and water was added dropwise. The mixture was stirred under ice-cooling and the precipitated crystals were collected by filtration to give the title compound.
¹H-NMR(300MHz, CDCl₃) δ:1.21(3H, t), 1.39(3H, t), 2.55(2H, t), 2.67(2H, t), 4.12(2H, q), 4.35(2H, q), 5.39(2H, s), 7.34-7.46(5H, m), 7.71(1H, s).

### Example 6

### Ethyl 3-(3-ethoxy-1H-pyrazol-4-yl)propionate

Benzyl 3-ethoxy-4-(3-ethoxy-3-oxopropyl)-1H-pyrazole-1-carboxylate (0.5 g) and 10% palladium carbon (0.05 g) were added to ethanol (5 mL) and the mixture was vigorously stirred under a hydrogen atmosphere for 2 hrs. Palladium carbon was filtered off and ethanol was evaporated to give the title compound as a colorless oil.
¹H-NMR(300MHz, CDCl₃) δ:1.24(3H, m), 1.38(3H, m), 2.55(2H, m), 2.67(2H, m), 4.12(2H, m), 4.25(2H, m), 7.17(1H, s), 8.47(1H, bs).

### Example 7

### Ethyl 3-(3-ethoxy-1-{4-[(2-phenyl-1,3-thiazol-4-yl)methoxy]benzyl}-1H-pyrazol-4-yl)propionate (hereinafter sometimes to be abbreviated as compound A)

Ethyl 3-(3-ethoxy-1H-pyrazol-4-yl)propionate (2 g) and 4-{[4-(chloromethyl)phenoxy]methyl}-2-phenyl-1,3-thiazole (3 g) were added to N,N-dimethylformamide (12 mL) and the mixture was cooled to near 5°C. Sodium tert-butoxide (1.2 g) was added and the mixture was stirred at room temperature for 2 hrs. Ethanol and dilute hydrochloric acid were added to the reaction mixture and the precipitated crystals were collected by filtration. The obtained crystals were recrystallized from isopropanol and n-heptane to give the title compound as pale-yellow white crystals.
¹H-NMR(300MHz, CDCl₃) δ:1.18(3H, m), 1.36(3H, m), 2.50 (2H, m), 2.64(2H, m), 4.08(2H, m), 4.22(2H, m), 5.00(2H, s), 5.25(2H, s), 6.93-6.99(3H, m), 7.13(2H, m), 7.29(1H, s), 7.45(3H, m), 7.92-7.96(2H, m).

### Example 8

The following compounds can be synthesized in the same manner as in Example 7.
Ethyl 3-[3-ethoxy-1-[4-(5-methyl-2-phenyl-4-oxazolylmethoxy)benzyl]-1H-pyrazol-4-yl]propionate (hereinafter sometimes to be abbreviated as compound B);
Ethyl 3-[3-ethoxy-1-[4-[2-(2-furyl)-5-methyl-4-oxazolylmethoxy]benzyl]-1H-pyrazol-4-yl]propionate (hereinafter sometimes to be abbreviated as compound C);
Ethyl 3-[3-ethoxy-1-[3-methoxy-4-(5-methyl-2-phenyl-4-oxazolylmethoxy)benzyl]-1H-pyrazol-4-yl]propionate (hereinafter sometimes to be abbreviated as compound D);
Ethyl 3-[3-ethoxy-1-[4-[2-(2-furyl)-5-methyl-4-oxazolylmethoxy]-3-methoxybenzyl]-1H-pyrazol-4-yl]propionate (hereinafter sometimes to be abbreviated as compound E);
Ethyl 3-[3-ethoxy-1-[3-methoxy-4-[5-methyl-2-(2-thienyl)-4-oxazolylmethoxy]benzyl]-1H-pyrazol-4-yl]propionate (hereinafter sometimes to be abbreviated as compound F);
Ethyl 3-[3-ethoxy-1-[3-methoxy-4-(3-pyridylmethoxy)benzyl]-1H-pyrazol-4-yl]propionate (hereinafter sometimes to be abbreviated as compound G);
Ethyl 3-[3-ethoxy-1-[2-(5-methyl-2-phenyl-4-oxazolylmethoxy)-5-pyridylmethyl]-1H-pyrazol-4-yl]propionate (hereinafter sometimes to be abbreviated as compound H);
Ethyl 3-[3-ethoxy-2-[4-(2-phenyl-4-oxazolylmethoxy)benzyl]-1H-pyrazol-4-yl]propionate (hereinafter sometimes to be abbreviated as compound I);
Ethyl 3-[3-ethoxy-1-[4-(3-pyridylmethoxy)benzyl]-1H-pyrazol-4-yl]propionate (hereinafter sometimes to be abbreviated as compound J);
Ethyl 3-[3-ethoxy-1-[4-[5-methyl-2-(2-thienyl)-4-oxazolylmethoxy]benzyl]-1H-pyrazol-4-yl]propionate (hereinafter sometimes to be abbreviated as compound K);
Ethyl 3-[3-ethoxy-1-[4-[2-(5-ethyl-2-pyridyl)ethoxy]benzyl]-1H-pyrazol-4-yl]propionate (hereinafter sometimes to be abbreviated as compound L);
Ethyl 3-[3-ethoxy-1-[2-[4-(5-methyl-2-phenyl-4-oxazolylmethoxy)phenyl]ethyl]-1H-pyrazol-4-yl]propionate (hereinafter sometimes to be abbreviated as compound M);
Ethyl 3-[3-ethoxy-1-[2-(5-methyl-2-phenyl-4-oxazolylmethoxy)benzyl]-1H-pyrazol-4-yl]propionate (hereinafter sometimes to be abbreviated as compound N);
Ethyl 3-[3-ethoxy-1-[3-(5-methyl-2-phenyl-4-oxazolylmethoxy)benzyl]-1H-pyrazol-4-yl]propionate (hereinafter sometimes to be abbreviated as compound O)

### Example 9

To a mixture of ethyl 3-(3-ethoxy-1H-pyrazol-4-yl)propionate (318 mg), 5-chloromethyl-2-(5-methyl-2-phenyl-4-oxazolylmethoxy)pyridine (472 mg) and N,N-dimethylformamide (10 ml) was added sodium hydride (60% in oil, 60.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound H (651 mg, yield 88%) was obtained as a colorless oil from a fraction eluted with ethyl acetate-hexane (1:1, volume ratio).
NMR(CDCl₃)δ:1.21 (3H, t, J=7.2 Hz) , 1.36 (3H, t, J=7.0 Hz), 2.47-2.55 (5H, m), 2.61-2.69 (2H, m), 4.09 (2H, q, J=7.2 Hz), 4.21 (2H, q, J=7.0 Hz), 4.98 (2H, s), 5.28 (2H, s), 6.77 (1H, d, J=8.4 Hz), 6.97 (1H, s), 7.39-7.46 (4H, m), 7.98-8.04 (3H, m).

### Example 10

To a mixture of ethyl 3-(3-ethoxy-1H-pyrazol-4-yl)propionate (318 mg), 4-(4-chloromethylphenoxymethyl)-2-phenyloxazole (450 mg) and N,N-dimethylformamide (10 ml) was added sodium hydride (60% in oil, 60.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The obtained colorless crystals were collected by filtration to give compound I (616 mg, yield 86%). The crystals were recrystallized from ethyl acetate-hexane. melting point: 80-81°C.

### Example 11

To a mixture of ethyl 3-(3-ethoxy-1H-pyrazol-4-yl)propionate (415 mg), 3-(4-chloromethylphenoxymethyl)pyridine (554 mg) and N,N-dimethylformamide (10 ml) was added sodium hydride (60% in oil 80.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound J (340 mg, yield 55%) was obtained as a colorless oil from a fraction eluted with ethyl acetate-hexane (1:1, volume ratio).
NMR(CDCl₃)δ:1.21 (3H, t, J=7.2 Hz), 1.36 (3H, t, J=7.0 Hz), 2.47-2.55 (2H, m), 2.61,-2.69 (2H, m), 4.09 (2H, q, J=7.2 Hz), 4.22 (2H, q, J=7.0 Hz), 5.00 (2H, s), 5.07 (2H, s), 6.92 (2H, d, J=8.8 Hz), 6. 9.3 (1H, s), 7.14 (2H, d, J=8.8 Hz), 7.32 (1H, dd, J=7.4, 4.8 Hz), 7.77 (1H, dt, J=7.4, 2.0 Hz), 8.59 (1H, dd, J=4.8, 2.0 Hz), 8.68 (1H, d, J=2.0 Hz).

### Example 12

To a mixture of ethyl 3-(3-ethoxy-1H-pyrazol-4-yl)propionate (318 mg), 2-[2-(4-chloromethylphenoxy)ethyl]-5-ethylpyridine (414 mg) and N,N-dimethylformamide (10 ml) was added sodium hydride (60% in oil, 60.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound L (520 mg, yield 77%) was obtained as a colorless oil from a fraction eluted with ethyl acetate-hexane (1:1, volume ratio).
NMR(CDCl₃)δ:1.20 (3H, t, J=7.0 Hz), 1.24 (3H, t, J=7.6 Hz), 1.36 (3H, t, J=7.0 Hz), 2.46-2.54 (2H, m), 2.57-2.68 (4H, m), 3.22 (2H, t, J=6.6 Hz), 4.08 (2H, q, J=7.0 Hz), 4.12 (2H, q, J=7.0 Hz), 4.33 (2H, t, J=6.6 Hz), 4.97 (2H, s), 6.85 (2H, d, J=8.6 Hz), 6.90 (1H, s), 7.11 (2H, d, J=8.6 Hz), 7.18 (1H, d, J=7.8 Hz), 7.45 (1H, dd, J=7.8, 2.0 Hz), 8.39 (1H, d, J=2.0 Hz).

### Example 13

To a mixture of ethyl 3-(3-ethoxy-1H-pyrazol-4-yl)propionate (509 mg), 4-(2-chloromethylphenoxymethyl)-5-methyl-2-phenyloxazole (753 mg) and N,N-dimethylformamide (10 ml) was added sodium hydride (60% in oil, 96.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound N (1.09 g, yield 93%) was obtained as a colorless oil from a fraction eluted with ethyl acetate-hexane (1:2, volume ratio).
NMR(CDCl₃)δ:1.20 (3H, t, J=7.2 Hz), 1.34 (3H, t, J=7.2 Hz), 2.39 (3H, s), 2.46-2.54 (2H, m), 2.60-2.68 (2H, m), 4.08 (2H, q, J=7.2 Hz), 4.20 (2H, q, J=7.2 Hz), 5.03 (2H, s), 5.11 (2H, s), 6.91-6.93 (2H, m), 7.01-7.06 (2H, m), 7.22-7.31 (1H, m), 7.41-7.48 (3H, m), 7.98-8.05 (2H, m).

### Example 14

To a mixture of ethyl 3-(3-ethoxy-1H-pyrazol-4-yl)propionate (509 mg), 4-(3-chloromethylphenoxymethyl)-5-methyl-2-phenyloxazole (753 mg) and N,N-dimethylformamide (10 ml) was added sodium hydride (60% in oil, 96.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound O (809 mg, yield 69%) was obtained as a colorless oil from a fraction eluted with ethyl acetate-hexane (1:3; volume ratio).
NMR(CDCl₃)δ:1.21 (3H, t, J=7.2 Hz), 1.36 (3H, t, J=7.2 Hz), 2.42 (3H, s), 2.48-2.55 (2H, m), 2.61-2.69 (2H, m), 4.09 (2H, q, J=7.2 Hz), 4.22 (2H, q, J=7.2 Hz), 4.95 (2H, s), 5.04 (2H, s), 6.74-6.83 (2H, m), 6.91-6.97 (2H, m), 7.25 (1H, t, J=7.8 Hz), 7.42-7.45 (3H, m), 7.99-8.04 (2H, m).

### Example 15

### 3-(3-Ethoxy-1-{4-[(2-phenyl-1,3-thiazol-4-yl)methoxy]benzyl}-1H-pyrazol-4-yl)propanoic acid (hereinafter sometimes to be abbreviated as compound P)

Ethyl 3-(3-ethoxy-1-{4-[(2-phenyl-1,3-thiazol-4-yl)methoxy]benzyl}-2H-pyrazol-4-yl)propionate (3 g) was suspended in ethanol (15 mL) and 1N aqueous sodium hydroxide solution (12.2 mL) was added. The mixture was stirred at 40°C for 2 hrs. Water and toluene were added to the reaction mixture for extraction. The aqueous layer was neutralized with dilute hydrochloric acid and the precipitated crystals were collected by filtration. The obtained crystals were recrystallized from aqueous ethanol to give the title compound as white crystals.
¹H-NMR(300MHz, DMSO-d₆) δ:1.27 (3H, m) , 2.38-2.51 (4H, m), 4.10(2H, m), 5.00(2H, s), 5.21 (2H, s), 7.02(2H, m), 7.16 (2H, m), 7.49-7.54(3H, m), 7.75(1H, s), 7.93-7.96(2H, m), 12.05(1H, bs).

### Example 16

The following compounds can be synthesized in the same manner as in Example 15 and using the compound obtained in Example 8.
3-[3-Ethoxy-1-[4-(5-methyl-2-phenyl-4-oxazolylmethoxy)benzyl]-1H-pyrazol-4-yl]propionic acid (hereinafter sometimes to be abbreviated as compound Q);
3-[3-Ethoxy-1-[4-[2-(2-furyl)-5-methyl-4-oxazolylmethoxy]benzyl]-1H-pyrazol-4-yl]propionic acid (hereinafter sometimes to be abbreviated as compound R);
3-[3-Ethoxy-1-[3-methoxy-4-(5-methyl-2-phenyl-4-oxazolylmethoxy)benzyl]-1H-pyrazol-4-yl]propionic acid (hereinafter sometimes to be abbreviated as compound S);
3-[3-Ethoxy-1-[4-[2-(2-furyl)-5-methyl-4-oxazolylmethoxy]-3-methoxybenzyl]-1H-pyrazol-4-yl]propionic acid (hereinafter sometimes to be abbreviated as compound T);
3-[3-Ethoxy-1-[3-methoxy-4-[5-methyl-2-(2-thienyl)-4-oxazolylmethoxy]benzyl]-1H-pyrazol-4-yl]propionic acid (hereinafter sometimes to be abbreviated as compound U);
3-[3-Ethoxy-3-[3-methoxy-4-(3-pyridylmethoxy)benzyl]-1H-pyrazol-4-yl]propionic acid (hereinafter sometimes to be abbreviated as compound V);
3-[3-Ethoxy-1-[2-(5-methyl-2-phenyl-4-oxazolylmethoxy)-5-pyridylmethyl]-1H-pyrazol-4-yl]propionic acid (hereinafter sometimes to be abbreviated as compound W);
3-[3-Ethoxy-1-[4-(2-phenyl-4-oxazolylmethoxy)benzyl]-1H-pyrazol-4-yl]propionic acid (hereinafter sometimes to be abbreviated as compound X);
3-[3-Ethoxy-1-[4-(3-pyridylmethoxy)benzyl]-1H-pyrazol-4-yl]propionic acid (hereinafter sometimes to be abbreviated as compound Y);
3-[3-Ethoxy-1-[4-[5-methyl-2-(2-thienyl)-4-oxazolylmethoxy]benzyl]-1H-pyrazol-4-yl]propionic acid (hereinafter sometimes to be abbreviated as compound Z);
3-[3-Ethoxy-1-[4-[2-(5-ethyl-2-pyridyl)ethoxy]benzyl]-1H-pyrazol-4-yl]propionic acid (hereinafter sometimes to be abbreviated as compound AA);
3-[3-Ethoxy-1-[2-[4-(5-methyl-2-phenyl-4-oxazolylmethoxy)phenyl]ethyl]-1H-pyrazol-4-yl]propionic acid (hereinafter sometimes to be abbreviated as compound AB) ;
3-[3-Ethoxy-1-[2-(5-methyl-2-phenyl-4-oxazolylmethoxy)benzyl]-1H-pyrazol-4-yl]propionic acid (hereinafter sometimes to be abbreviated as compound AC);
3-[3-Ethoxy-1-[3-(5-methyl-2-phenyl-4-oxazolylmethoxy)benzyl]-1H-pyrazol-4-yl]propionic acid (hereinafter sometimes to be abbreviated as compound AD)

### Example 17

A mixture of ethyl 3-[3-ethoxy-1-[4-(5-methyl-2-phenyl-4-oxazolylmethoxy)benzyl]-1H-pyrazol-4-yl]propionate (441 mg), 1N aqueous sodium hydroxide solution (2 ml), tetrahydrofuran (4 ml) and ethanol (4 ml) was stirred at room temperature for 2 hrs. 1N Hydrochloric acid (2 ml) was added and the mixture was extracted with ethyl acetate. The ethyl acetate layer was washed with saturated brine, dried (MgSO₄) and concentrated. The obtained colorless crystals were collected by filtration to give compound Q (328 mg, yield 79%). The crystals were recrystallized from ethanol-hexane. melting point: 96-97°C.

### Example 18

A mixture of ethyl 3-[3-ethoxy-1-[4-[2-(2-furyl)-5-methyl-4-oxazolylmethoxy]benzyl]-2H-pyrazol-4-yl]propionate (381 mg), 1N aqueous sodium hydroxide solution (2 ml), tetrahydrofuran (4 ml) and ethanol (4 ml) was stirred at room temperature for 4 hrs. 1N Hydrochloric acid (2 ml) was added and the mixture was extracted with ethyl acetate. The ethyl acetate layer was washed with saturated brine, dried (MgSO₄) and concentrated. The obtained colorless crystals were collected by filtration to give compound R (327 mg, yield 91%). The crystals were recrystallized from ethanol-hexane. melting point: 129-130°C.

### Example 19

A mixture of ethyl 3-[3-ethoxy-1-[3-methoxy-4-(5-methyl-2-phenyl-4-oxazolylmethoxy)benzyl]-1H-pyrazol-4-yl]propionate (660 mg), 1N aqueous sodium hydroxide solution (3 ml), tetrahydrofuran (6 ml) and ethanol (6 ml) was stirred at room temperature for 3 hrs. 1N Hydrochloric acid (3 ml) was added and the mixture was extracted with ethyl acetate. The ethyl acetate layer was washed with saturated brine, dried (MgSO₄) and concentrated. The obtained colorless crystals were collected by filtration to give compound S (510 mg, yield 82%). The crystals were recrystallized from ethanol-hexane. melting point: 122-123°C.

### Example 20

A mixture of ethyl 3-[3-ethoxy-1-[4-[2-(2-furyl)-5-methyl-4-oxazolylmethoxy]-3-methoxybenzyl]-1H-pyrazol-4-yl]propionate (561 mg), 1N aqueous sodium hydroxide solution (2.5 ml), tetrahydrofuran (5 ml) and ethanol (5 ml) was stirred at room temperature for 2 hrs. 1N Hydrochloric acid (2.5 ml) was added and the mixture was extracted with ethyl acetate. The ethyl acetate layer was washed with saturated brine, dried (MgSO₄) and concentrated. The obtained colorless crystals were collected by filtration to give compound T (506 mg, yield 96%). The crystals were recrystallized from ethanol-hexane. melting point: 133-134°C.

### Example 21

A mixture of ethyl 3-[3-ethoxy-1-[3-methoxy-4-[5-methyl-2-(2-thienyl)-4-oxazolylmethoxy)benzyl]-1H-pyrazol-4-yl]propionate (499 mg), 1N aqueous sodium hydroxide solution (2 ml), tetrahydrofuran (4 ml) and ethanol (4 ml) was stirred at room temperature for 3 hrs. 1N Hydrochloric acid (2 ml) was added and the mixture was extracted with ethyl acetate. The ethyl acetate layer was washed with saturated brine, dried (MgSO₄) and concentrated. The obtained colorless crystals were collected by filtration to give compound U (392 mg, yield 83%). The crystals were recrystallized from ethanol-hexane. melting point: 123-124°C.

### Example 22

A mixture of ethyl 3-[3-ethoxy-1-[3-methoxy-4-(3-pyridylmethoxy)benzyl]-1H-pyrazol-4-yl]propionate (527 mg), 1N aqueous sodium hydroxide solution (2.5 ml), tetrahydrofuran (5 ml) and ethanol (5 ml) was stirred at room temperature for 2 hrs. 1N Hydrochloric acid (2.5 ml) was added and the mixture was extracted with ethyl acetate. The ethyl acetate layer was washed with saturated brine, dried (MgSO₄) and concentrated. The obtained colorless crystals were collected by filtration to give compound V (381 mg, yield 77%). The crystals were recrystallized from ethanol-hexane. melting point: 124-125°C.

### Example 23

A mixture of ethyl 3-[3-ethoxy-1-[2-(5-methyl-2-phenyl-4-oxazolylmethoxy)-5-pyridylmethyl]-1H-pyrazol-4-yl]propionate (638 mg), 1N aqueous sodium hydroxide solution (2.5 ml), tetrahydrofuran (5 ml) and ethanol (5 ml) was stirred at room temperature for 3 hrs. 1N Hydrochloric acid (2.5 ml) was added and the mixture was extracted with ethyl acetate. The ethyl acetate layer was washed with saturated brine, dried (MgSO₄) and concentrated. The obtained colorless crystals were collected by filtration to give compound W (495 mg, yield 82%). The crystals were recrystallized from ethanol-hexane. melting point: 143-144°C.

### Example 24

A mixture of ethyl 3-[3-ethoxy-1-[4-(2-phenyl-4-oxazolylmethoxy)benzyl]-1H-pyrazol-4-yl]propionate (523 mg), 1N aqueous sodium hydroxide solution (2.5 ml), tetrahydrofuran (5 ml) and ethanol (5 ml) was stirred at room temperature for 3 hrs. 1N Hydrochloric acid (2.5 ml) was added and the mixture was extracted with ethyl acetate. The ethyl acetate layer was washed with saturated brine, dried (MgSO₄) and concentrated. The obtained colorless crystals were collected by filtration to give compound X (456 mg, yield 93%). The crystals were recrystallized from ethanol-hexane. melting point: 135-136°C.

### Example 25

A mixture of ethyl 3-[3-ethoxy-1-[4-(3-pyridylmethoxy)benzyl]-1H-pyrazol-4-yl]propionate (340 mg), 1N aqueous sodium hydroxide solution (2 ml), tetrahydrofuran (4 ml) and ethanol (4 ml) was stirred at room temperature for 2 hrs. 1N Hydrochloric acid (2 ml) was added and the mixture was extracted with ethyl acetate. The ethyl acetate layer was washed with saturated brine, dried (MgSO₄) and concentrated. The obtained colorless crystals were collected by filtration to give compound Y (260 mg, yield 82%) . The crystals were recrystallized from ethanol-hexane. melting point: 120-121°C.

### Example 26

To a mixture of ethyl 3-(3-ethoxy-1H-pyrazol-4-yl)propionate (300 mg), 4-(4-chloromethylphenoxymethyl)-5-methyl-2-(2-thienyl)oxazole (450 mg) and N,N-dimethylformamide (5 ml) was added sodium hydride (60% in oil, 70.0 mg) at 0°C and the mixture was stirred at room temperature for 30 min. The reaction mixture was poured into water and the mixture was extracted with ethyl acetate. The ethyl acetate layer was washed with water and then with saturated brine, dried (MgSO₄) and concentrated. The residue 'was applied to silica gel column chromatography and a colorless oil was obtained from a fraction eluted with ethyl acetate-hexane (1:2, volume ratio). A mixture of the obtained oil, 1N aqueous sodium hydroxide solution (5 ml), tetrahydrofuran (5 ml) and ethanol (5 ml) was stirred overnight at room temperature. 1N Hydrochloric acid (5 ml) was added and the mixture was extracted with ethyl acetate. The ethyl acetate layer was washed with saturated brine, dried (MgSO₄) and concentrated. The obtained colorless crystals were collected by filtration to give compound Z (460 mg, yield 70%). The crystals were recrystallized from acetone-hexane. melting point: 156-157°C.

### Example 27

A mixture of ethyl 3-[3-ethoxy-1-[4-[2-(5-ethyl-2-pyridyl)ethoxy]benzyl]-1H-pyrazol-4-yl]propionate (519 mg), 1N aqueous sodium hydroxide solution (2.5 ml), tetrahydrofuran (5 ml) and ethanol (5 ml) was stirred at room temperature for 3 hrs. 1N Hydrochloric acid (2.5 ml) was added and the mixture was extracted with ethyl acetate. The ethyl acetate layer was washed with saturated brine, dried (MgSO₄) and concentrated. The obtained colorless crystals were collected by filtration to give compound AA (228 mg, yield 47%). The crystals were recrystallized from ethanol-hexane. melting point: 89-90°C.

### Example 28

A mixture of ethyl 3-[3-ethoxy-1-[2-[4-(5-methyl-2-phenyl-4-oxazolylmethoxy)phenyl]ethyl]-1H-pyrazol-4-yl]propionate (1.08 g), 1N aqueous sodium hydroxide solution (4.2 ml), tetrahydrofuran (3 ml) and ethanol (3 ml) was stirred at room temperature for 2 hrs. 1N Hydrochloric acid (5 ml) was added and the mixture was extracted with ethyl acetate. The ethyl acetate layer was washed with saturated brine, dried (MgSO₄) and concentrated. The obtained colorless crystals were collected by filtration to give compound AB (880 mg, yield 88%). The crystals were recrystallized from ethyl acetate-hexane. melting point: 110-111°C.

### Example 29

A mixture of ethyl 3-[3-ethoxy-1-[2-(5-methyl-2-phenyl-4-oxazolylmethoxy)benzyl]-1H-pyrazol-4-yl]propionate (1.09 g), 1N aqueous sodium hydroxide solution (5 ml), tetrahydrofuran (10 ml) and ethanol (10 ml) was stirred at room temperature for 2 hrs. 1N Hydrochloric acid (5 ml) was added and the mixture was extracted with ethyl acetate. The ethyl acetate layer was washed with saturated brine, dried (MgSO₄) and concentrated. The obtained colorless crystals were collected by filtration to give compound AC (834 mg, yield 82%). The crystals were recrystallized from ethanol-hexane. melting point: 127-128°C.

### Example 30

A mixture of ethyl 3-[3-ethoxy-1-[3-(5-methyl-2-phenyl-4-oxazolylmethoxy)benzyl]-1H-pyrazol-4-yl]propionate (808 mg), 1N aqueous sodium hydroxide solution (4 ml), tetrahydrofuran (8 ml) and ethanol (8 ml) was stirred at room temperature for 3 hrs. 1N Hydrochloric acid (4 ml) was added and the mixture was extracted with ethyl acetate. The ethyl acetate layer was washed with saturated brine, dried (MgSO₄) and concentrated. The residue was applied to silica gel column chromatography and compound AD (709 mg, yield 93%) was obtained as a colorless oil from a fraction eluted with ethyl acetate-methanol (5:1, volume ratio).
NMR(CDCl₃)δ: 1.35 (3H, t, J=7.0 Hz), 2.48 (3H, s), 2.67 (4H, s), 4.21 (2H, q, J=7.0 Hz), 4.96 (2H, s), 5.11 (2H, s), 6.42 (1H, s), 6.84-6.91 (2H, m), 7.19 (1H, s), 7.26 (1H, d, J=8.0 Hz), 7.43-7.47 (3H, m), 7.94-7.99 (2H, m).

### Example 31

To a solution of ethyl 3-[3-hydroxy-1-[4-(5-methyl-2-phenyl-4-oxazolylmethoxy)benzyl]-1H-pyrazol-4-yl]propionate (462 mg) in N,N-dimethylformamide (10 ml) was added sodium hydride (60% in oil, 40.0 mg) at 0°C and the mixture was stirred at room temperature for 30 min. To the reaction mixture was added iodoethane (0.240 ml) and the mixture was stirred at room temperature for 1 hr. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound B (452 mg, yield 92%) was obtained as a colorless oil from a fraction eluted with ethyl acetate-hexane (1:1, volume ratio).
NMR(CDCl₃)δ: 1.21 (3H, t, J=7.2 Hz), 1.36 (3H, t, J=7.0 Hz), 2.43 (3H, s) , 2.47-2.55 (2H, m) , 2.61-2.69 (2H, m), 4.09 (2H, q, J=7.2 Hz), 4.23 (2H, q, J=7.0 Hz), 4.98 (2H, s), 5.00 (2H, s), 6.93 (1H, s), 6.97 (2H, d, J=8.8 Hz), 7.14 (2H, d, J=8.8 Hz), 7.40-7.47 (3H, m), 7.97-8.06 (2H, m).

### Example 32

To a solution of ethyl 3-[1-[4-[2-(2-furyl)-5-methyl-4-oxazolylmethoxy]benzyl]-3-hydroxy-1H-pyrazol-4-yl]propionate (452 mg) in N,N-dimethylformamide (10 ml) was added sodium hydride (60% in oil, 40.0 mg) at 0°C and the mixture was stirred at room temperature for 30 min. To the reaction mixture was added iodoethane (0.240 ml) and the mixture was stirred at room temperature for 1 hr. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound C (348 mg, yield 73%) was obtained as a colorless oil from a fraction eluted with ethyl acetate-hexane (1:1, volume ratio).
NMR(CDCl₃)δ: 1.21 (3H, t, J=7.0 Hz) , 1.36 (3H, t, J=7.0 Hz), 2.47 (3H, s), 2.48-2.55 (2H, m), 2.61-2.69 (2H, m), 4.09 (2H, q, J=7.0 Hz), 4.22 (2H, q, J=7.0 Hz), 4.97 (2H, s), 5.00 (2H, s), 6.52 (1H, dd, J=3.6, 2.0 Hz), 6.92-6.99 (4H, m), 7.13 (2H, d, J=8.8 Hz), 7.54 (1H, dd, J=2.0, 0.6 Hz).

### Example 33

To a solution of ethyl 3-[3-ethoxy-1-(4-hydroxy-3-methoxybenzyl)-1H-pyrazol-4-yl]propionate (505 mg) in N,N-dimethylformamide (10 ml) was added sodium hydride (60% in oil, 58.0 mg) at 0°C and the mixture was stirred at room temperature for 30 min. To the reaction mixture was added 4-chloromethyl-5-methyl-2-phenyloxazole (301 mg) and the mixture was stirred at 60°C for 1 hr. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound D (661 mg, yield 88%) was obtained as a colorless oil from a fraction eluted with ethyl acetate-hexane (1:1, volume ratio).
NMR(CDCl₃)δ: 1.22 (3H, t, J=7.2 Hz), 1.37 (3H, t, J=7.2 Hz), 2.41 (3H, s), 2.47-2.55 (2H, m), 2.61-2.69 (2H, m), 3.82 (3H, s), 4.09 (2H, q, J=7.2 Hz), 4.23 (2H, q, J=7.2 Hz), 5.00 (2H, s), 5.04 (2H, s), 6.72 (1H, dd, J=8.2, 2.2 Hz), 6.76 (1H, d, J=2.2 Hz), 6.95 (1H, s), 7.00 (1H, d, J=8.2 Hz), 7.40-7.46 (3H, m), 7.98-8.03 (2H, m).

### Example 34

To a solution of ethyl 3-[3-ethoxy-1-(4-hydroxy-3-methoxybenzyl)-1H-pyrazol-4-yl]propionate (505 mg) in N,N-dimethylformamide (10 ml) was added sodium hydride (60% in oil, 58.0 mg) at 0°C and the mixture was stirred at room temperature for 30 min. To the reaction mixture was added 4-chloromethyl-2-(2-furyl)-5-methyloxazole (573 mg) and the mixture was stirred at room temperature for 1 hr. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound E (564 mg, yield 76%) was obtained as a yellow oil from a fraction eluted with ethyl acetate-hexane (1:1, volume ratio).
NMR(CDCl₃)δ: 1.21 (3H, t, J=7.2 Hz), 1.37 (3H, t, J=7.2 Hz), 2.40 (3H, s), 2.47-2.55 (2H, m), 2.61-2.69 (2H, m), 3.82 (3H, s), 4.09 (2H, q, J=7.2 Hz), 4.23 (2H, q, J=7.2 Hz), 4.99 (2H, s), 5.03 (2H, s), 6.52 (1H, dd, J=3.6, 1.8 Hz), 6.71 (1H, dd, J=8.2, 2.0 Hz), 6.75 (1H, d, J=2.0 Hz), 6.94 (1H, s), 6.96 (1H, d, J=8.2 Hz), 6.96 (1H, dd, J=3.6, 0.8 Hz), 7.53 (1H, dd, J=1.8, 0.8 Hz).

### Example 35

To a solution of ethyl 3-[3-ethoxy-1-(4-hydroxy-3-methoxybenzyl)-1H-pyrazol-4-yl]propionate (505 mg) in N,N-dimethylformamide (10 ml) was added sodium hydride (60% in oil, 58.0 mg) at 0°C and the mixture was stirred at room temperature for 30 min. To the reaction mixture was added 4-chloromethyl-5-methyl-2-(2-thienyl)oxazole (310 mg) and the mixture was stirred at room temperature for 1 hr. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound F (500 mg, yield 66%) was obtained as a colorless oil from a fraction eluted with ethyl acetate-hexane (1:1, volume ratio).
NMR(CDCl₃)δ: 1.21 (3H, t, J=7.2 Hz), 1.37 (3H, t, J=7.2 Hz), 2.39 (3H, s), 2.47-2.55 (2H, m), 2.61-2.69 (2H, m), 3.82 (3H, s), 4.09 (2H, q, J=7.2 Hz), 4.23 (2H, q, J=7.2 Hz), 4.99 (2H, s), 5.01 (2H, s), 6.71 (1H, dd, J=8.0, 2.2 Hz), 6.75 (1H, d, J=2.2 Hz), 6.94 (1H, s), 6.96 (1H, d, J=8.0 Hz), 7.09 (1H, dd, J=4.8, 3.6 Hz), 7.39 (1H, dd, J=4.8, 1.2 Hz), 7.62 (1H, dd, J=3.6, 1.2 Hz).

### Example 36

A mixture of ethyl 3-[3-ethoxy-1-(4-hydroxy-3-methoxybenzyl)-1H-pyrazol-4-yl]propionate (505 mg), 3-picolyl chloride hydrochloride (476 mg), potassium carbonate (601 mg) and N,N-dimethylformamide (10 ml) was stirred overnight at room temperature. The reaction mixture was poured into water and the mixture was extracted with ethyl acetate. The ethyl acetate layer was washed with water and then with saturated brine, dried (MgSO₄) and concentrated. The residue was applied to silica gel column chromatography and compound G (531 mg, yield 83%) was obtained as a colorless oil from a fraction eluted with ethyl acetate.
NMR(CDCl₃)δ:1.21 (3H, t, J=7.0 Hz), 1.37 (3H, t, J=7.0 Hz), 2.47-2.55 (2H, m), 2.61-2.69 (2H, m), 3.84 (3H, s), 4.09 (2H, q, J=7.0 Hz), 4.22 (2H, q, J=7.0 Hz), 4.99 (2H, s), 5.13 (2H, s), 6.68 (1H, dd, J=8.0, 2.2 Hz), 6.77 (1H, d, J=2.2 Hz), 6.84 (1H, d, J=8.0 Hz), 6.95 (1H, s), 7.31 (1H, dd, J=8.0, 4.8 Hz), 7.79 (1H, dt, J=8.0, 1.8 Hz), 8.57 (1H, d, J=4.8 Hz), 8.67 (1H, s).

### Example 37

### Ethyl 3-(3-hydroxy-1-{4-[(2-phenyl-1,3-thiazol-4-yl)methoxy]benzyl}-1H-pyrazol-4-ylpropionate

To a suspension of 4-(2-phenyl-1,3-thiazol-4-yl)methoxy]benzyl alcohol (2.97 g) in toluene (30 mL) was added one drop of N,N-dimethylformamide, and thionyl chloride (3.6 mL) was further added dropwise. The mixture was stirred at 95°C for 2 hrs. The solvent was evaporated and toluene (10 mL) was added to the residue. Toluene was evaporated to give 4-{[4-(chloromethyl)phenoxy]methyl}-2-phenyl-1,3-thiazole. The residue was dissolved in N,N-dimethylformamide (30 mL) and ethyl 3-hydroxy-1H-pyrazol-4-propionate (1.66 g) and potassium carbonate (4.15 g) were added. The mixture was stirred at 45-50°C for 8 hrs. To the reaction mixture was added water (30 mL) and the mixture was extracted with ethyl acetate. The organic layer was washed with 20% brine and activated carbon (200 mg) was added. The mixture was stirred at room temperature for 30 min. Then, activated carbon was filtered off and the solvent was evaporated. To the obtained oil was added methanol-diisopropyl ether (1:1, 8 mL) and the mixture was stirred at 40°C for 1 hr., at room temperature for 1 hr. and under ice-cooling for 1 hr. The precipitated crystals were collected by filtration to give the title compound as a pale-brown powder.
¹H-NMR(300MHz, CDCl₃)δ: 1.23(3H, t, J=7.1Hz), 2.52-2.57(2H, m),2.63-2.68(2H, m), 4.10(2H, q, J=7.1Hz), 4.96(2H, s), 5.26(2H, s), 6.92(1H, s), 6.98(2H, d, J=8.5Hz), 7.21(2H, d, J=8.5Hz), 7.31(1H, s), 7.43-7.48 (3H, m), 7.94-7.97 (2H, m).

| Elemental analysis value: for C₂₅H₂₅N₃O₄S | | | | |
|---|---|---|---|---|
| Calculated; | C, 64.78 | H, 5.44 | N, 9.06 | S, 6.92 |
| Found; | C, 64.79 | H, 5.58 | N, 9.17 | S, 6.85 |

### Example 38

To a solution of ethyl 3-(3-hydroxy-1-{4-[(2-phenyl-1,3-thiazol-4-yl)methoxy]benzyl}-1H-pyrazol-4-ylpropionate (4.64 g) in N,N-dimethylformamide (20 mL) were added ethyl iodide (0.96 mL) and sodium tert-butoxide (980 mg) under ice-cooling. The mixture was stirred at room temperature for 2 hrs. to give ethyl 3-(3-ethoxy-1-{4-[(2-phenyl-1,3-thiazol-4-yl)methoxy]benzyl}-1H-pyrazol-4-yl)propionate. To the reaction mixture was added dropwise 4N aqueous sodium hydroxide solution (5 ml) and the mixture was stirred at room temperature for 4 hrs. 6N Hydrochloric acid was added to adjust to pH 5-4, and the mixture was extracted with ethyl acetate. The extract was washed with 20% brine and the solvent was evaporated. To the obtained solid was added 2-propanol (40 mL) and dissolved with heating. The mixture was stirred at room temperature for 2 hrs. and under ice-cooling for 2 hrs. The precipitated crystals were collected by filtration to give compound P as a pale-brown powder. Recrystallization was conducted from 10% aqueous methanol.
¹H-NMR(300MHz, CDCl₃)δ: 1.35(3H, t, J=7.0Hz), 2.54-2.60(2H, m),2.65-2.68(2H, m), 4.22(2H, q, J=7.0Hz), 5.00(2H, s), 5.25(2H, s), 6.94-6.98 (3H, m), 7.13(2H, d, J=8.6Hz), 7.29(1H, s), 7.41-7.46(3H, m), 7.93-7.96(2H, m).

| Elemental analysis value: for C₂₅H₂₅N₃O₄S | | | | |
|---|---|---|---|---|
| Calculated; | C, 64.78 | H, 5.44 | N, 9.06 | S, 6.92 |
| Found; | C, 64.82 | H, 5.37 | N, 9.13 | S, 6.93 |

### Formulation Example 1: production of film tablet

### [Production of coating agent]

Hydroxypropylmethyl cellulose 2910 (TC-5) (358.8 g) and macrogol 6000 (polyethylene glycol 6000) (72 g) were dissolved in purified water (4320 g). Titanium oxide (48 g) and yellow ferric oxide (1.2 g) were dispersed in the resulting solution to produce a coating agent.

### [Production of naked tablet]

Compound I (8 g), lactose (3568 g) and corn starch (440 g) are uniformly mixed in a fluidized bed granulation dryer (FD-5S, POWREX) and granulated while spraying an aqueous solution of hydroxypropyl cellulose (HPC-L) (132 g) therein, which is followed by drying in the fluidized bed granulation dryer.

The obtained granules are pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) are added to the obtained granulated powder (3526 g) and admixed in a tumbler mixer (TM-15, SHOWA KAGAKU KIKAI KOUSAKUSHO) to give granules for tableting. The resulting granules are punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent is sprayed on the resulting naked tablets in DRIACOATER (DRC-500, POWREX) to give 26000 film-coated tablets of the following formulation containing 0.2 mg of compound I per tablet.

### Formulation of naked tablet (composition per tablet):

| | | |
|---|---|---|
| 1) | compound I | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet) :

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01 mg |
| | Total | 114.0 mg |

### Formulation Example 2: Production of film tablet

In the same manner as in Formulation Example 1 except that the amount of compound I and lactose to be used is set to 40 g and 3536 g, respectively, 26000 film-coated tablets of the following formulation containing 1 mg of compound I per tablet are obtained.

### Formulation of naked tablet (composition per tablet):

| | | |
|---|---|---|
| 1) | compound I | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 3: Production of film tablet

In the same manner as in Formulation Example 1 except that the amount of compound I and lactose to be used is set to 160 g and 3416 g, respectively, 26000 film-coated tablets of the following formulation containing 4 mg of compound I per tablet are obtained.

### Formulation of naked tablet (composition per tablet):

| | | |
|---|---|---|
| 1) | compound I | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 4: Production of film tablet

In the same manner as in Formulation Example 1 except that the amount of compound I and lactose to be used is set to 640 g and 2936 g, respectively, 26000 film-coated tablets of the following formulation containing 16 mg of compound I per tablet are obtained.

### Formulation of naked tablet (composition per tablet):

| | | |
|---|---|---|
| 1) | compound I | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet) :

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 5: Production of film tablet

### [Production of coating agent]

Hydroxypropylmethyl cellulose 2910 (TC-5) (430.8 g) was dissolved in purified water (4320 g). Titanium oxide (48 g) and yellow ferric oxide (1.2 g) were dispersed in the resulting solution to produce a coating agent.

### [Production of naked tablet]

Compound N (8 g), lactose (3568 g) and corn starch (440 g) are uniformly mixed in a fluidized bed granulation dryer (FD-5S, POWREX) and granulated while spraying an aqueous solution of hydroxypropyl cellulose (HPC-L) (132 g) therein, which is followed by drying in the fluidized bed granulation dryer.

The obtained granules are pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) are added to the obtained granulated powder (3526 g) and admixed in a tumbler mixer (TM-15, SHOWA KAGAKU KIKAI KOUSAKUSHO) to give granules for tableting. The resulting granules are punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent is sprayed on the resulting naked tablets in DRIACOATER (DRC-500, POWREX) to give 26000 film-coated tablets of the following formulation containing 0.2 mg of compound N per tablet.

### Formulation of naked tablet (composition per tablet):

| | | |
|---|---|---|
| 1) | compound N | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 6: Production of film tablet

In the same manner as in Formulation Example 5 except that the amount of compound N and lactose to be used is set to 40 g and 3536 g, respectively, 26000 film-coated tablets of the following formulation containing 1 mg of compound N per tablet are obtained.

### Formulation of naked tablet (composition per tablet):

| | | |
|---|---|---|
| 1) | compound N | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 7: Production of film tablet

In the same manner as in Formulation Example 5 except that the amount of compound N and lactose to be used is set to 160 g and 3416 g, respectively, 26000 film-coated tablets of the following formulation containing 4 mg of compound N per tablet are obtained.

### Formulation of naked tablet (composition per tablet):

| | | |
|---|---|---|
| 1) | compound N | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01 mg |
| | Total | 114.0 mg |

### Formulation Example 8: Production of film tablet

In the same manner as in Formulation Example 5 except that the amount of compound N and lactose to be used is set to 640 g and 2936 g, respectively, 26000 film-coated tablets of the following formulation containing 16 mg of compound N per tablet are obtained.

### Formulation of naked tablet (composition per tablet):

| | | |
|---|---|---|
| 1) | compound N | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01 mg |
| | Total | 114.0 mg |

### Formulation Example 9: Production of film tablet

### [Production of coating agent]

Hydroxypropylmethyl cellulose 2910 (TC-5) (358.8 g) and macrogol 6000 (polyethylene glycol 6000) (72 g) were dissolved in purified water (4320 g). Titanium oxide (48 g) and yellow ferric oxide (1.2 g) were dispersed in the resulting solution to produce a coating agent.

### [Production of naked tablet]

Compound P (8 g), lactose (3568 g) and corn starch (440 g) were uniformly mixed in a fluidized bed granulation dryer (FD-5S, POWREX) and granulated while spraying an aqueous solution of hydroxypropyl cellulose (HPC-L) (132 g) therein, which was followed by drying in the fluidized bed granulation dryer.

The obtained granules were pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) were added to the obtained granulated powder (3526 g) and admixed in a tumbler mixer (TM-15, SHOWA KAGAKU KIKAI KOUSAKUSHO) to give granules for tableting. The resulting granules were punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent was sprayed on the resulting naked tablets in DRIACOATER (DRC-500, POWREX) to give 260.00 film-coated tablets of the following formulation containing 0.2 mg of compound P per tablet.

### Formulation of naked tablet (composition per tablet):

| | | |
|---|---|---|
| 1) | compound P | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 10: Production of film tablet

In the same manner as in Formulation Example 9 except that the amount of compound P and lactose to be used was set to 40 g and 3536 g, respectively, 26000 film-coated tablets of the following formulation containing 1 mg of compound P per tablet were obtained.

### Formulation of naked tablet (composition per tablet):

| | | |
|---|---|---|
| 1) | compound P | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 11: Production of film tablet

In the same manner as in Formulation Example 9 except that the amount of compound P and lactose to be used was set to 160 g and 3416 g, respectively, 26000 film-coated tablets of the following formulation containing 4 mg of compound P per tablet were obtained.

### Formulation of naked tablet (composition per tablet):

| | | |
|---|---|---|
| 1) | compound P | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 12: Production of film tablet

In the same manner as in Formulation Example 9 except that the amount of compound P and lactose to be used was set to 640 g and 2936 g, respectively, 26000 film-coated tablets of the following formulation containing 16 mg of compound P per tablet were obtained.

### Formulation of naked tablet (composition per tablet):

| | | |
|---|---|---|
| 1) | compound P | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 13: Production of film tablet

### [Production of coating agent]

Hydroxypropylmethyl cellulose 2910 (TC-5) (430.8 g) was dissolved in purified water (4320 g). Titanium oxide (48 g) and yellow ferric oxide (1.2 g) were dispersed in the resulting solution to produce a coating agent.

### [Production of naked tablet]

Compound P (8 g), lactose (3568 g) and corn starch (440 g) were uniformly mixed in a fluidized bed granulation dryer (FD-5S, POWREX) and granulated while spraying an aqueous solution of hydroxypropyl cellulose (HPC-L) (132 g) therein, which was followed by drying in the fluidized bed granulation dryer.

The obtained granules were pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) were added to the obtained granulated powder (3526 g) and admixed in a tumbler mixer (TM-15, SHOWA KAGAKU KIKAI KOUSAKUSHO) to give granules for tableting. The resulting granules were punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent was sprayed on the resulting naked tablets in DRIACOATER (DRC-500, POWREX) to give 26000 film-coated tablets of the following formulation containing 0.2 mg of compound P per tablet.

### Formulation of naked tablet (composition per tablet):

| | | |
|---|---|---|
| 1) | compound P | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 14: Production of film tablet

In the same manner as in Formulation Example 13 except that the amount of compound P and lactose to be used was set to 40 g and 3536 g, respectively, 26000 film-coated tablets of the following formulation containing 1 mg of compound P per tablet were obtained.

### Formulation of naked tablet (composition per tablet):

| | | |
|---|---|---|
| 1) | compound P | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 15: Production of film tablet

In the same manner as in Formulation Example 13 except that the amount of compound P and lactose to be used was set to 160 g and 3416 g, respectively, 26000 film-coated tablets of the following formulation containing 4 mg of compound P per tablet were obtained.

### Formulation of naked tablet (composition per tablet):

| | | |
|---|---|---|
| 1) | compound P | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 16: Production of film tablet

In the same manner as in Formulation Example 13 except that the amount of compound P and lactose to be used was set to 640 g and 2936 g, respectively, 26000 film-coated tablets of the following formulation containing 16 mg of compound P per tablet were obtained.

### Formulation of naked tablet (composition per tablet):

| | | |
|---|---|---|
| 1) | compound P | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 17: Production of film tablet

### [Production of coating agent]

Hydroxypropylmethyl cellulose 2910 (TC-5) (358.8 g) and macrogol 6000 (polyethylene glycol 6000) (72 g) were dissolved in purified water (4320 g). Titanium oxide (48 g) and yellow ferric oxide (1.2 g) were dispersed in the resulting solution to produce a coating agent.

### [Production of naked tablet]

Compound R (8 g), lactose (3568 g) and corn starch (440 g) are uniformly mixed in a fluidized bed granulation dryer (FD-5S, POWREX) and granulated while spraying an aqueous solution of hydroxypropyl cellulose (HPC-L) (132 g) therein, which is followed by drying in the fluidized bed granulation dryer.

The obtained granules are pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) are added to the obtained granulated powder (3526 g) and admixed in a tumbler mixer (TM-15, SHOWA KAGAKU KIKAI KOUSAKUSHO) to give granules for tableting. The resulting granules are punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent is sprayed on the resulting naked tablets in DRIACOATER (DRC-500, POWREX) to give 26000 film-coated tablets of the following formulation containing 0.2 mg of compound R per tablet.

### Formulation of naked tablet (composition per tablet):

| | | |
|---|---|---|
| 1) | compound R | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01 mg |
| | Total | 114.0 mg |

### Formulation Example 18: Production of film tablet

In the same manner as in Formulation Example 17 except that the amount of compound R and lactose to be used is set to 40 g and 3536 g, respectively, 26000 film-coated tablets of the following formulation containing 1 mg of compound R per tablet are obtained.

### Formulation of naked tablet (composition per tablet):

| | | |
|---|---|---|
| 1) | compound R | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01 mg |
| | Total | 114.0 mg |

### Formulation Example 19: Production of film tablet

In the same manner as in Formulation Example 17 except that the amount of compound R and lactose to be used is set to 160 g and 3416 g, respectively, 26000 film-coated tablets of the following formulation containing 4 mg of compound R per tablet are obtained.

### Formulation of naked tablet (composition per tablet):

| | | |
|---|---|---|
| 1) | compound R | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01mg |
| | Total | 114.0 mg |

### Formulation Example 20: Production of film tablet

In the same manner as in Formulation Example 17 except that the amount of compound R and lactose to be used is set to 640 g and 2936 g, respectively, 26000 film-coated tablets of the following formulation containing 16 mg of compound R per tablet are obtained.

### Formulation of naked tablet (composition per tablet):

| | | |
|---|---|---|
| 1) | compound R | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 2.99 mg |
| 3) | macrogol 6000 | 0.6 mg |
| 4) | titanium oxide | 0.4 mg |
| 5) | yellow ferric oxide | 0.01 mg |
| | Total | 114.0 mg |

### Formulation Example 21: Production of film tablet

### [Production of coating agent]

Hydroxypropylmethyl cellulose 2910 (TC-5) (430.8 g) was dissolved in purified water (4320 g). Titanium oxide (48 g) and yellow ferric oxide (1.2 g) were dispersed in the resulting solution to produce a coating agent.

### [Production of naked tablet]

Compound Z (8 g), lactose (3568 g) and corn starch (440 g) are uniformly mixed in a fluidized bed granulation dryer (FD-5S, POWREX) and granulated while spraying an aqueous solution of hydroxypropyl cellulose (HPC-L) (132 g) therein, which is followed by drying in the fluidized bed granulation dryer.

The obtained granules are pulverized with a power mill (P-3, SHOWA KAGAKU KIKAI KOUSAKUSHO) using a 1.5 mmφ punching screen to give a granulated powder.

Croscarmellose sodium (187 g) and magnesium stearate (27.2 g) are added to the obtained granulated powder (3526 g) and admixed in a tumbler mixer (TM-15, SHOWA KAGAKU KIKAI KOUSAKUSHO) to give granules for tableting. The resulting granules are punched with a rotary tableting machine (Correct 12HUK, KIKUSUI SEISAKUSHO LTD.) using a 6.5 mmφ punch at the weight of 110 mg (punching pressure 9.5 KN/punch) to give naked tablets.

### [Production of film-coated tablet]

The aforementioned coating agent is sprayed on the resulting naked tablets in DRIACOATER (DRC-500, POWREX) to give 26000 film-coated tablets of the following formulation containing 0.2 mg of compound Z per tablet.

### Formulation of naked tablet (composition per tablet):

| | | |
|---|---|---|
| 1) | compound Z | 0.2 mg |
| 2) | lactose | 89.2 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01 mg |
| | Total | 114.0 mg |

### Formulation Example 22: Production of film tablet

In the same manner as in Formulation Example 21 except that the amount of compound Z and lactose to be used is set to 40 g and 3536 g, respectively, 26000 film-coated tablets of the following formulation containing 1 mg of compound Z per tablet are obtained.

### Formulation of naked tablet (composition per tablet) :

| | | |
|---|---|---|
| 1) | compound Z | 1.0 mg |
| 2) | lactose | 88.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01mg |
| | Total | 114.0 mg |

### Formulation Example 23: Production of film tablet

In the same manner as in Formulation Example 21 except that the amount of compound Z and lactose to be used is set to 160 g and 3416 g, respectively, 26000 film-coated tablets of the following formulation containing 4 mg of compound Z per tablet are obtained.

### Formulation of naked tablet (composition per tablet):

| | | |
|---|---|---|
| 1) | compound Z | 4.0 mg |
| 2) | lactose | 85.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01 mg |
| | Total | 114.0 mg |

### Formulation Example 24: Production of film tablet

In the same manner as in Formulation Example 21 except that the amount of compound Z and lactose to be used is set to 640 g and 2936 g, respectively, 26000 film-coated tablets of the following formulation containing 16 mg of compound Z per tablet are obtained.

### Formulation of naked tablet (composition per tablet):

| | | |
|---|---|---|
| 1) | compound Z | 16.0 mg |
| 2) | lactose | 73.4 mg |
| 3) | corn starch | 11.0 mg |
| 4) | croscarmellose sodium | 5.5 mg |
| 5) | hydroxypropyl cellulose | 3.3 mg |
| 6) | magnesium stearate | 0.8 mg |
| | Total | 110.0 mg |

### Formulation of film tablet (composition per tablet):

| | | |
|---|---|---|
| 1) | naked tablet (film component) | 110.0 mg |
| 2) | hydroxypropylmethyl cellulose 2910 | 3.59 mg |
| 3) | titanium oxide | 0.4 mg |
| 4) | yellow ferric oxide | 0.01 mg |
| | Total | 114.0 mg |

### Industrial Applicability

According to the production method of the present invention, a compound useful as a pharmaceutical agent such as a therapeutic agent for diabetes and the like or a synthetic intermediate therefor can be produced conveniently and in a high yield.

In addition, the compound of the present invention is useful as a pharmaceutical agent such as a therapeutic agent for diabetes and the like or a synthetic intermediate therefor.

## Claims

1. A production method of a compound represented by the formula (I) 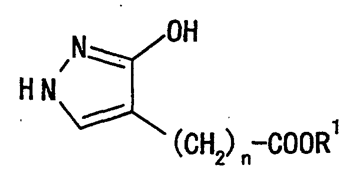 wherein n is an integer of 1 to 4 and R¹ is a hydrocarbon group optionally having substituents, or a salt thereof, which comprises reacting a compound represented by the formula (II) 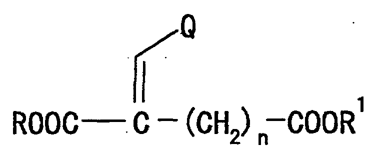 wherein Q is a hydroxy group optionally having substituents or an amino group optionally having substituents, R is a hydrocarbon group optionally having substituents and other symbols are as defined above, or a salt thereof with hydrazine or a salt thereof.

2. The production method of claim 1, wherein Q is a hydroxy group.

3. The production method of claim 1, wherein n is 2.

4. The production method of claim 1, wherein the hydrocarbon group optionally having substituents for R or R¹ is an alkyl group having 1 to 8 carbon atoms.

5. A compound represented by the formula (I) 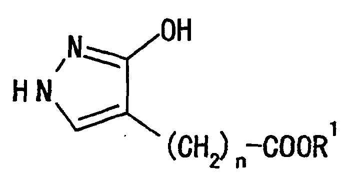 wherein n is an integer of 1 to 4 and R¹ is a hydrocarbon group optionally having substituents, or a salt thereof.

6. A production method of a compound represented by the formula (III) 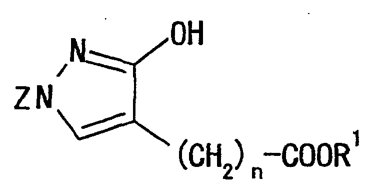 wherein Z is an acyl group, n is an integer of 1 to 4 and R¹ is a hydrocarbon group optionally having substituents, or a salt thereof, which comprises subjecting a compound represented by the formula (I) 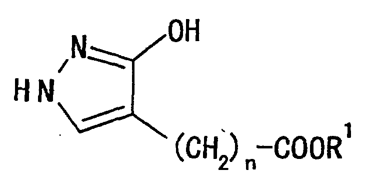 wherein the symbols in the formula are as defined above, or a salt thereof to an acylation reaction.

7. A compound represented by the formula (III) 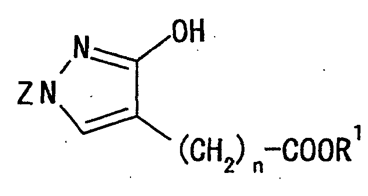 wherein Z is an acyl group, n is an integer of 1 to 4 and R¹ is a hydrocarbon group optionally having substituents, or a salt thereof.

8. A production method of a compound represented by the formula (IV) 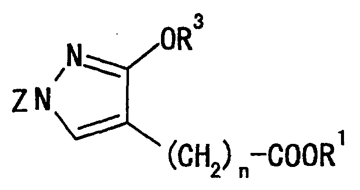 wherein R³ is an alkyl group, Z is an acyl group, n is an integer of 1 to 4 and R¹ is a hydrocarbon group optionally having substituents, or a salt thereof, which comprises subjecting a compound represented by the formula (III) 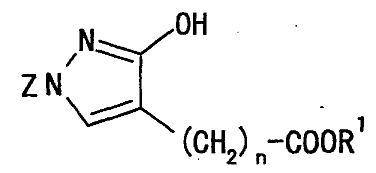 wherein the symbols in the formula are as defined above, or a salt thereof to an alkylation reaction.

9. A compound represented by the formula (IV) 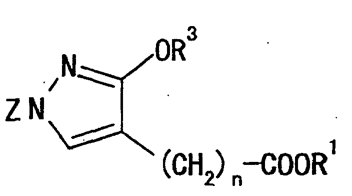 wherein Z is an acyl group, n is an integer of 1 to 4, R¹ is a hydrocarbon group optionally having substituents and R³ is an alkyl group, or a salt thereof.

10. A production method of a compound represented by the formula (V) 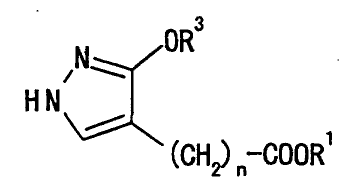 wherein n is an integer of 1 to 4, R¹ is a hydrocarbon group optionally having substituents and R³ is an alkyl group, or a salt thereof, which comprises subjecting a compound represented by the formula (IV) 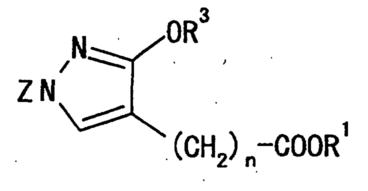 wherein Z is an acyl group and other symbols are as defined above, or a salt thereof to a deacylation reaction.

11. A production method of a compound represented by the formula (VII) 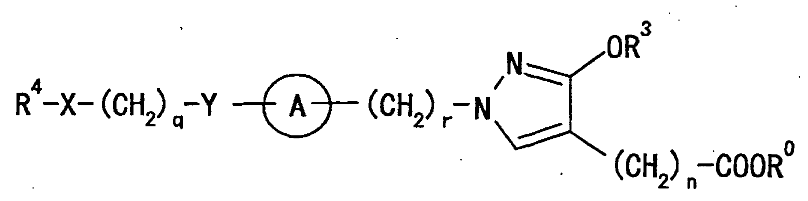 wherein R⁴ is a hydrocarbon group optionally having substituents or a heterocyclic group optionally having substituents,
X is a bond, an oxygen atom, a sulfur atom, -CO-, -CS-, -CR⁵(OR⁶)- or -NR⁷- wherein R⁵ and R⁷ are each a hydrogen atom or a hydrocarbon group optionally having substituents, R⁶ is a hydrogen atom or a hydroxyl-protecting group;
q is an integer of 0 to 3;
Y is an oxygen atom, a sulfur atom, -SO-, -SO₂-, -NR⁸-, -CONR⁸- or -NR⁸CO- wherein R⁸ is a hydrogen atom or a hydrocarbon group optionally having substituents;
ring A is an aromatic ring optionally further having 1 to 3 substituents;
r is an integer of 1 to 8;
R³ is an alkyl group;
n is an integer of 1 to 4; and
R⁰ is a hydrogen atom or a hydrocarbon group optionally having substituents, or a salt thereof, which comprises reacting a compound represented by the formula (II) 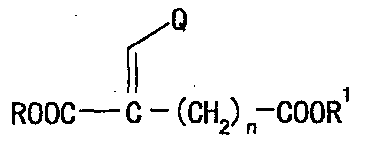 wherein Q is a hydroxy group optionally having substituents or an amino group optionally having substituents, and R and R¹ are the same or different and each is a hydrocarbon group optionally having substituents, or a salt thereof with hydrazine or a salt thereof to give a compound represented by the formula (I) 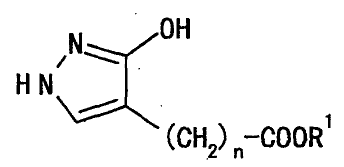 wherein the symbols in the formula are as defined above, or a salt thereof, subjecting the compound or a salt thereof to an acylation reaction to give a compound represented by the formula (III) 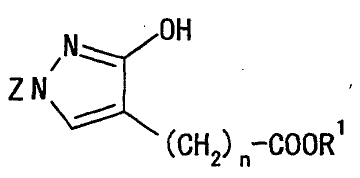 wherein Z is an acyl group and other symbols are as defined above, or a salt thereof, subjecting the compound or a salt thereof to an alkylation reaction to give a compound represented by the formula (IV) 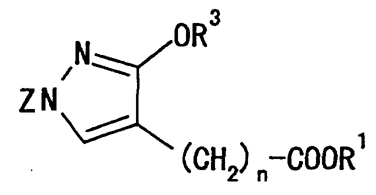 wherein the symbols in the formula are as defined above, or a salt thereof, subjecting the compound or a salt thereof to a deacylation reaction to give a compound represented by the formula (V) 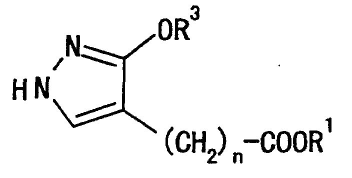 wherein the symbols in the formula are as defined above, or a salt thereof, reacting the compound or a salt thereof with a compound represented by the formula (VI) 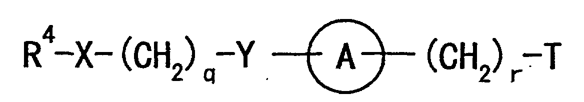 wherein T is a leaving group and other symbols are as defined above, or a salt thereof, and conducting a hydrolysis reaction where necessary.

12. A production method of a compound represented by the formula (VII) 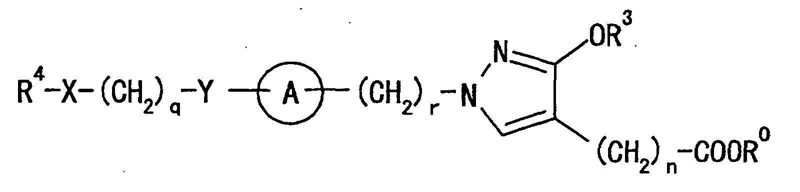 wherein R⁴ is a hydrocarbon group optionally having substituents or a heterocyclic group optionally having substituents,
X is a bond, an oxygen atom, a sulfur atom, -CO-, -CS-, -CR⁵(OR⁶)- or -NR⁷- wherein R⁵ and R⁷ are each a hydrogen atom or a hydrocarbon group optionally having substituents, R⁶ is a hydrogen atom or a hydroxyl-protecting group;
q is an integer of 0 to 3;
Y is an oxygen atom, a sulfur atom, -SO-, -SO₂-, -NR⁸-, -CONR⁸- or -NR⁸CO- wherein R⁸ is a hydrogen atom or a hydrocarbon group optionally having substituents;
ring A is an aromatic ring optionally further having 1 to 3 substituents;
r is an integer of 1 to 8;
n is an integer of 1 to 4;
R⁰ is a hydrogen atom or a hydrocarbon group optionally having substituents, or a salt thereof, which comprises reacting a compound represented by the formula (II) 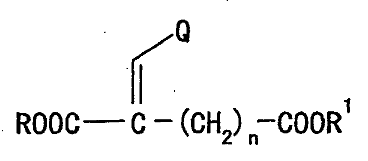 wherein Q is a hydroxy group optionally having substituents or an amino group optionally having substituents, and R and R¹ are the same or different and each is a hydrocarbon group optionally having substituents, or a salt thereof, with hydrazine or a salt thereof to give a compound represented by the formula (I) 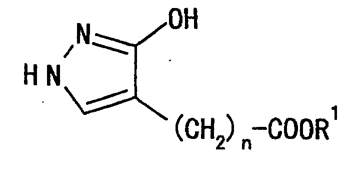 wherein the symbols in the formula are as defined above, or a salt thereof, reacting the compound or a salt thereof with a compound represented by the formula (VI) 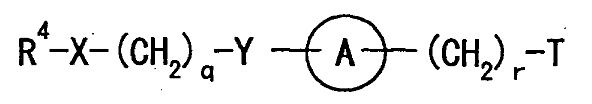 wherein T is a leaving group and other symbols are as defined above, or a salt thereof to give a compound represented by the formula (VIII) 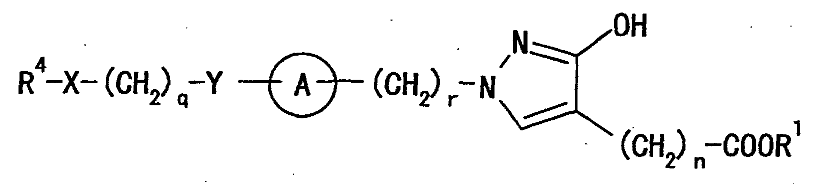 wherein the symbols in the formula are as defined above, or a salt thereof, subjecting the compound or a salt thereof to an alkylation reaction, and conducting a hydrolysis reaction where necessary.
